# EUROPEAN PATENT SPECIFICATION

(11) **EP 3 989 986 B1**
(45) Date of publication and mention of the grant of the patent: **09.07.2025**
(21) Application number: 21772208.1
(22) Date of filing: 17.03.2021
(51) Int. Cl.: A61K 35/17, A61K 39/395, A61K 47/64

(54) **COMBINED CHIMERIC ANTIGEN RECEPTOR TARGETING CD19 AND CD20 AND APPLICATIONS THEREOF**
GEGEN CD19 UND CD20 GERICHTETER KOMBINIERTER CHIMÄRER ANTIGEN-REZEPTOR UND ANWENDUNGEN DAVON
RÉCEPTEUR D'ANTIGÈNE CHIMÈRE COMBINÉ CIBLANT CD19 ET CD20 ET SES APPLICATIONS

(30) Priority: 17.03.2020 CN 202010188038; 18.05.2020 US 202016877069; 17.08.2020 WO PCT/CN2020/109645; 26.02.2021 US 202163154032 P
(43) Date of publication of application: 04.05.2022
(73) Proprietor: AbelZeta Inc., Rockville, MD 20850 (US)
(72) Inventor: YAO, Yihong, Hong Kong (HK); HUANG, Jiaqi, Hong Kong (HK); YAO, Xin, Hong Kong (HK); ZHU, Shigui, Hong Kong (HK); LI, Yanfeng., Hong Kong (HK); WEI, Yutian., Hong Kong (HK)
(74) Representative: Cabinet Becker et Associés
(86) International application number: PCT/US2021/022779
(87) International publication number: WO 2021/188681

(56) References cited:
- US-A1- 2017 355 767
- US-A1- 2017 368 098
- ZAH EUGENIA ET AL: "T Cells Expressing CD19/CD20 Bispecific Chimeric Antigen Receptors Prevent Antigen Escape by Malignant B Cells", CANCER IMMUNOLOGY RESEARCH, vol. 4, no. 6, 1 June 2016 (2016-06-01), US, pages 498 - 508, XP055835189, ISSN: 2326-6066, Retrieved from the Internet <URL:https://www.ncbi.nlm.nih.gov/pmc/articles/PMC4933590/pdf/nihms781209.pdf> DOI: 10.1158/2326-6066.CIR-15-0231
- HUI ZHOU ET AL: "The efficacy and safety of anti-CD19/CD20 chimeric antigen receptor- T cells immunotherapy in relapsed or refractory B-cell malignancies:a meta-analysis", BMC CANCER, vol. 18, no. 1, 26 September 2018 (2018-09-26), XP055765246, DOI: 10.1186/s12885-018-4817-4
- DINA SCHNEIDER ET AL: "A tandem CD19/CD20 CAR lentiviral vector drives on-target and off-target antigen modulation in leukemia cell lines", JOURNAL FOR IMMUNOTHERAPY OF CANCER, BIOMED CENTRAL LTD, LONDON, UK, vol. 5, no. 1, 16 May 2017 (2017-05-16), pages 1 - 17, XP021245145, DOI: 10.1186/S40425-017-0246-1
- ALEXANDRA MARTYNISZYN ET AL: "CD20-CD19 Bispecific CAR T Cells for the Treatment of B-Cell Malignancies", HUMAN GENE THERAPY, vol. 28, no. 12, 1 December 2017 (2017-12-01), GB, pages 1147 - 1157, XP055589293, ISSN: 1043-0342, DOI: 10.1089/hum.2017.126
- TONG CHUAN ET AL: "Optimized tandem CD19/CD20 CAR-engineered T cells in refractory/relapsed B cell lymphoma", BLOOD, 18 June 2020 (2020-06-18), US, XP055955333, ISSN: 0006-4971, DOI: 10.1182/blood.2020005278
- LIANG AIBIN ET AL: "Safety and efficacy of a novel anti-CD20/CD19 bi-specific CAR T-cell therapy (C-CAR039) in relapsed or refractory (r/r) B-cell non-Hodgkin lymphoma (B-NHL).", JOURNAL OF CLINICAL ONCOLOGY, vol. 39, no. 15_suppl, 20 May 2021 (2021-05-20), US, pages 2507 - 2507, XP055955198, ISSN: 0732-183X, Retrieved from the Internet <URL:http://dx.doi.org/10.1200/JCO.2021.39.15_suppl.2507> DOI: 10.1200/JCO.2021.39.15_suppl.2507
- ZAH EUGENIA, LIN MENG-YIN, SILVA-BENEDICT ANNE, JENSEN MICHAEL C., CHEN YVONNE Y.: "T Cells Expressing CD19/CD20 Bispecific Chimeric Antigen Receptors Prevent Antigen Escape by Malignant B Cells", CANCER IMMUNOLOGY RESEARCH, AMERICAN ASSOCIATION FOR CANCER RESEARCH, US, vol. 4, no. 6, 1 June 2016 (2016-06-01), US , pages 498 - 508, XP055835189, ISSN: 2326-6066, DOI: 10.1158/2326-6066.CIR-15-0231
- DATABASE PROTEIN ANONYMOUS: "bi-specific chimeric antigen receptor 20-19 Long, partial [synthetic construct]", XP055812059, retrieved from NCBI

## Description

### Technical field

The present invention relates to the field of biomedicine, and more particularly to a combined chimeric antigen receptor targeting CD19 and CD20 and applications thereof.

### Background

Malignant tumors of the blood system account for about 10% of human malignant tumors, and 95% of malignant tumors of the blood system are derived from B lymphocytes. Traditional chemotherapy and radiotherapy play an important role in the treatment of malignant tumors of the blood system. Some patients have significant effects, but it is difficult for most of the patients to be cured. New and effective treatments have been a hot topic in this field.

Adoptive T cell therapy has shown its powerful efficacy and bright prospect in the clinical treatment of malignant tumors. At present, it is regarded as one of the most promising methods for treating hematological tumors. CD19 is highly expressed on the surface of most B-cell malignancies. Multiple centers independently using Chimeric Antigen Receptor (CAR)-modified T cells to target recurrent, refractory malignant tumors of CD19-expressed B cell have achieved unprecedented success. At present, both of the CAR-T products approved by FDA are targeting CD19 antigen and their indications are also expanding, such as chronic lymphocytic leukemia. Although the efficacy of anti-CD19 CAR-T is outstanding, many studies have shown that there are also many problems with CD19 chimeric antigen receptor (CAR) T cell therapy. There are still some patients with poor treatment results and easy to relapse. The reasons for this include the susceptibility of tumor cells to antigen escape. For example, a recent experiment of CD19 CAR-cell therapy showed that 90% of patients achieved complete remission, but 11% of these patients eventually relapsed, and the relapsing patients were mainly patients with CD19-negative tumor. In particular, in a clinical trial carried out at the University of Pennsylvania School of Medicine using CART19 in the treatment of recurrent, refractory acute B-cell lymphoma (R/R B-ALL), up to 94% of patients achieved complete remission. Although the initial response rate of this clinical trial was high, nearly 40% of patients relapsed after 1 month of treatment which achieved complete remission, and more than 60% of relapsing patients had CD19-negative tumor cells escape. Antigen escape has been found in adoptive transfer specific T cell receptors expressing NY-ESO1 and cancer vaccines treating melanoma. Spontaneous mutation and selective expansion are the main reasons for antigen escape.

Therefore, there is an urgent need to develop methods for effectively treating tumors and preventing antigen escape.

### Summary of the invention

The present disclosure relates to a method for effectively treating tumors and preventing antigen escape.

It is herein provided a combined chimeric antigen receptor targeting CD19 and CD20 and preparation method thereof.

Specifically, the invention is as defined in the appended claims. The invention is directed to a bispecific chimeric antigen receptor (CAR) targeting CD19 and CD20, wherein the CAR comprises the amino acid sequence set forth in SEQ ID NO. 16.

A preparation method and activity identification of the modified T cell (CART-19/20) thereof are described. The present invention also provides the chimeric antigen receptor structure as defined herein for use in the treatment of CD19 and CD20 positive B cell lymphoma.

The present disclosure provides for an immune cell expressing the present bispecific CAR. The immune cell may be a T cell or a natural killer (NK) cell. The immune cell may be allogeneic or autologous.

Also encompassed by the present disclosure is a nucleic acid encoding the present bispecific CAR.

The present disclosure further provides for a vector comprising the present nucleic acid.

The present disclosure provides for a method of treating cancer. The method may comprise administering the immune cell to a subject in need thereof.

The cancer may be a hematologic cancer. The cancer may be a B-cell malignancy. The cancer may be Hodgkin's lymphoma, non-Hodgkin's lymphoma, leukemia, and/or multiple myeloma. The cancer may be acute myeloid leukemia (AML), multiple myeloma (MM), chronic lymphocytic leukemia (CLL), chronic myelogenous leukemia, acute lymphoblastic leukemia (ALL), diffuse large B cell lymphoma (DLBCL), or combinations thereof.

In a second aspect of the invention, it provides a nucleic acid molecule, encoding the chimeric antigen receptor of the first aspect of the invention.

In another preferred embodiment, the nucleic acid molecule is isolated.

In another preferred embodiment, the nucleotide sequence of the nucleic acid molecule is shown in SEQ ID NO: 17 or 18.

In a third aspect of the invention, it provides a vector, comprising the nucleic acid molecule of the second aspect of the invention.

In another preferred embodiment, the vector comprises DNA and RNA.

In another preferred embodiment, the vector is selected from the group consisting of plasmid, virus vector, transposon, and a combination thereof.

In another preferred embodiment, the vector comprises a DNA virus and a retrovirus vector.

In another preferred embodiment, the vector is selected from the group consisting of a lentiviral vector, an adenovirus vector, an adeno-associated virus vector, and a combination thereof.

In another preferred embodiment, the vector is a lentiviral vector.

In a fourth aspect of the invention, it provides a host cell, comprising the vector of the third aspect of the invention or having the exogenous nucleic acid molecule of the second aspect of the invention integrated into its genome or expressing the chimeric antigen receptor of the first aspect of the invention.

In another preferred embodiment, the cell is an isolated cell.

In another preferred embodiment, the cell is a genetically engineered cell.

In another preferred embodiment, the cell is a mammalian cell.

In another preferred embodiment, the cell is a CAR-T cell and/or a CAR-NK cell.

In another preferred embodiment, the cell targets both CD19 and CD20.

In a fifth aspect of the invention, it provides a method for preparing a CAR-T cell expressing the chimeric antigen receptor of the first aspect of the invention, wherein the method comprises the steps of: transducing the nucleic acid molecule of the second aspect of the invention or the vector of the third aspect of the invention into a T cell, thereby obtaining the CAR-T cell.

In another preferred embodiment, the method further comprises the step of detecting the function and effectiveness of the obtained CAR-T cell.

In a sixth aspect of the invention, it provides a preparation, comprising the chimeric antigen receptor of the first aspect of the invention, the nucleic acid molecule of the second aspect of the invention, the vector of the third aspect of the invention, or the host cell of the fourth aspect of the invention, and a pharmaceutically acceptable carrier, diluent or excipient.

In another preferred embodiment, the preparation is a liquid preparation.

In another preferred embodiment, the formulation of the preparation is an injection.

In another preferred embodiment, the preparation comprises the host cell of the fourth aspect of the invention, and the concentration of the host cell is 1×10³-1×10⁸ cells/ml, preferably 1×10⁴-1×10⁷ cells/ml.

It is further herein described the use of the chimeric antigen receptor of the first aspect of the invention, the nucleic acid molecule of the second aspect of the invention, the vector of the third aspect of the invention, or the host cell of the fourth aspect of the invention, for the preparation of a medicine or a formulation for preventing and/or treating tumor or cancer.

In another preferred embodiment, the tumor is selected from the group consisting of a hematological tumor, a solid tumor, and a combination thereof; preferably, the tumor is a hematological tumor.

In another preferred embodiment, the blood tumor is selected from the group consisting of acute myeloid leukemia (AML), multiple myeloma (MM), chronic lymphocytic leukemia (CLL), acute lymphoblastic leukemia (ALL), diffuse large B cell lymphoma (DLBCL), and a combination thereof.

In another preferred embodiment, the solid tumor is selected from the group consisting of gastric cancer, peritoneal metastasis of gastric cancer, liver cancer, leukemia, renal cancer, lung cancer, small intestine cancer, bone cancer, prostate cancer, colorectal cancer, breast cancer, large intestine cancer, cervical cancer, ovarian cancer, lymphoma, nasopharyngeal carcinoma, adrenal tumor, bladder tumor, non-small cell lung cancer (NSCLC), glioma, endometrial cancer, and a combination thereof.

It is also described a kit for the preparation of the cell of the fourth aspect of the invention, wherein the kit comprises a container, and the nucleic acid molecule of the second aspect of the invention or the vector of the third aspect of the invention located in the container.

It is also described the use of the cell of the fourth aspect of the invention, or the formulation of the sixth aspect of the invention for the prevention and/or treatment of cancer or tumor.

It is further described a method of treating a disease comprising administering an appropriate amount of the cell of the forth aspect of the invention, or the formulation of the sixth aspect of the invention, to a subject in need of treatment.

In another preferred embodiment, the disease is cancer or tumor.

The present disclosure provides for an immune cell expressing the present bispecific CAR. The immune cell may be a T cell or a natural killer (NK) cell.

Also encompassed by the present disclosure is a nucleic acid encoding the present bispecific CAR.

The present disclosure provides for a vector comprising the present nucleic acid.

The present disclosure also provides for a method of treating cancer. The method may comprise administering the present immune cell to a subject in need thereof.

The immune cell may be allogeneic or autologous.

The cancer may be a hematologic cancer. The cancer may be a B-cell malignancy. The cancer may be Hodgkin's lymphoma, non-Hodgkin's lymphoma, leukemia, and/or multiple myeloma. The cancer may be acute myeloid leukemia (AML), multiple myeloma (MM), chronic lymphocytic leukemia (CLL), chronic myelogenous leukemia, acute lymphoblastic leukemia (ALL), diffuse large B cell lymphoma (DLBCL), or combinations thereof.

In certain embodiments, the bispecific CAR may generate an area under the curve (AUC) ranging from about 0.5e+06 copies/µg genomic DNA (copies/gDNA) to about 4e+06 copies/gDNA, from about 0.5e+06 copies/µg genomic DNA (copies/gDNA) to about 3.5e+06 copies/gDNA, from about 1e+06 copies/µg genomic DNA (copies/gDNA) to about 3.5e+06 copies/gDNA, from about 1.2e+06 copies/µg genomic DNA (copies/gDNA) to about 3.2e+06 copies/gDNA, from about 0.8e+06 copies/µg genomic DNA (copies/gDNA) to about 3.2e+06 copies/gDNA, from about 1.6e+06 copies/µg genomic DNA (copies/gDNA) to about 3.2e+06 copies/gDNA, from about 1e+06 copies/µg genomic DNA (copies/gDNA) to about 2e+06 copies/gDNA, from about 0.6e+06 copies/µg genomic DNA (copies/gDNA) to about 1.8e+06 copies/gDNA, from about 3e+06 copies/µg genomic DNA (copies/gDNA) to about 3.2e+06 copies/gDNA, from about 0.5e+06 copies/µg genomic DNA (copies/gDNA) to about 1.7e+06 copies/gDNA, from about 2e+06 copies/µg genomic DNA (copies/gDNA) to about 3.2e+06 copies/gDNA, from about 1.5e+06 copies/µg genomic DNA (copies/gDNA) to about 2e+06 copies/gDNA, or from about 1e+06 copies/µg genomic DNA (copies/gDNA) to about 3.2e+06 copies/gDNA, in the blood of the subject in about 28 days after administration of the bispecific CAR to the subject.

In certain embodiments, the bispecific CAR generates a maximum plasma concentration (Cₘₐₓ) ranging from about 0.8e+05 copies/µg genomic DNA (copies/gDNA) to about 3.5e+05 copies/gDNA, from about 1e+05 copies/µg genomic DNA (copies/gDNA) to about 3.5e+05 copies/gDNA, from about 1e+05 copies/µg genomic DNA (copies/gDNA) to about 1.6e+05 copies/gDNA, from about 1e+05 copies/µg genomic DNA (copies/gDNA) to about 3.3e+05 copies/gDNA, from about 0.8e+05 copies/µg genomic DNA (copies/gDNA) to about 1.5e+05 copies/gDNA, from about 0.8e+05 copies/µg genomic DNA (copies/gDNA) to about 2e+05 copies/gDNA, from about 1e+05 copies/µg genomic DNA (copies/gDNA) to about 2e+05 copies/gDNA, from about 2e+05 copies/µg genomic DNA (copies/gDNA) to about 3e+05 copies/gDNA, from about 2e+05 copies/µg genomic DNA (copies/gDNA) to about 3.5e+05 copies/gDNA, from about 2e+05 copies/µg genomic DNA (copies/gDNA) to about 2.5e+05 copies/gDNA, or from about 1e+05 copies/µg genomic DNA (copies/gDNA) to about 3e+05 copies/gDNA, in the blood of the subject after administration of the bispecific CAR to the subject.

In certain embodiments, the bispecific CAR has a Tₘₐₓ (the time it takes the bispecific CAR to reach Cₘₐₓ) ranging from about 3 days to about 20 days, from about 4 days to about 18 days, from about 5 days to about 17 days, from about 6 days to about 16 days, from about 7 days to about 15 days, from about 9 days to about 15 days, from about 10 days to about 15 days, from about 10 days to about 14 days, from about 8 days to about 12 days, from about 6 days to about 14 days, from about 12 days to about 14 days, from about 8 days to about 11 days, from about 8 days to about 15 days, or from about 10 days to about 14 days.

### Description of Drawings

Figure 1 shows the structure of the combined chimeric antigen receptor targeting CD19 and CD20. The structure of the CAR comprises a signal peptide sequence ("SP"), an antigen recognition sequence, a hinge region, a transmembrane region, a co-stimulatory factor signal region, and a CD3ζ signaling region.

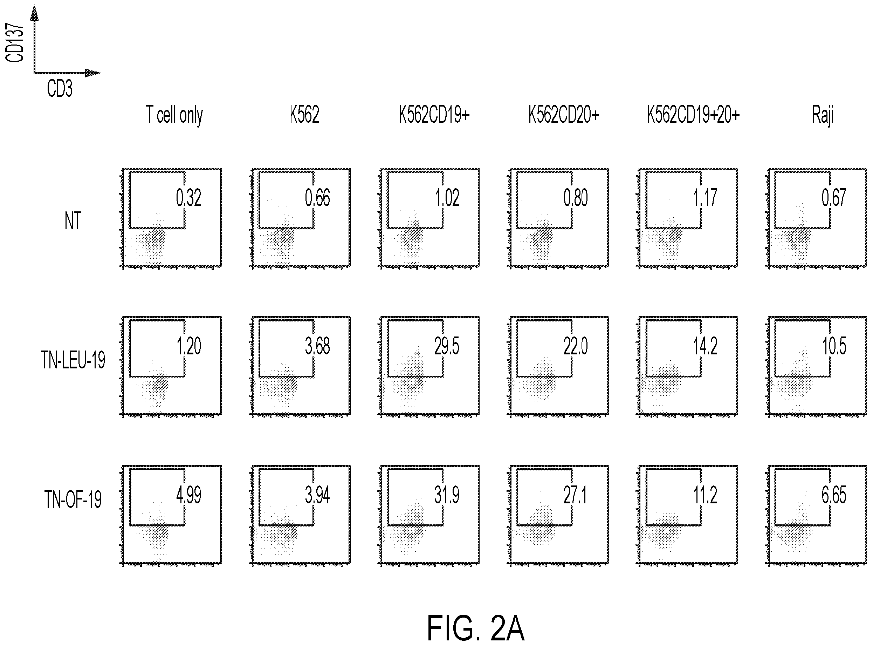

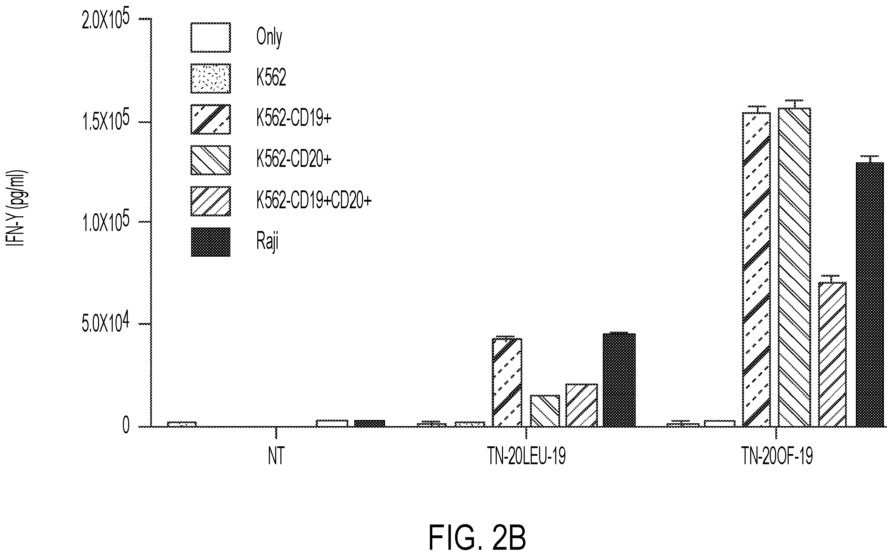

Figures 2A and 2B show the expression level of CD137 on the surface of T cell membrane (Fig. 2A) and the secretion level of IFNγ in the culture supernatant (Fig. 2B). Specifically, 1×10⁵ of CAR-T19/20s cells cultured 7 days were taken and cultured respectively with CD19-positive K562-CD19+ tumor cell line, CD20-positive K562-CD20+ tumor cell line, CD19 and CD20 double positive K562-CD19+CD20+ tumor cell line, RAJI tumor cell line that naturally expresses CD19 and CD20, and CD19 and CD20 double negative K562 tumor cell line, or without tumor cells, in 200 µl of GT-551 medium for 18h with a ratio of 1:1. Then the expression level of CD137 on the surface of T cell membrane and the secretion level of IFNγ in the culture supernatant were detected respectively.

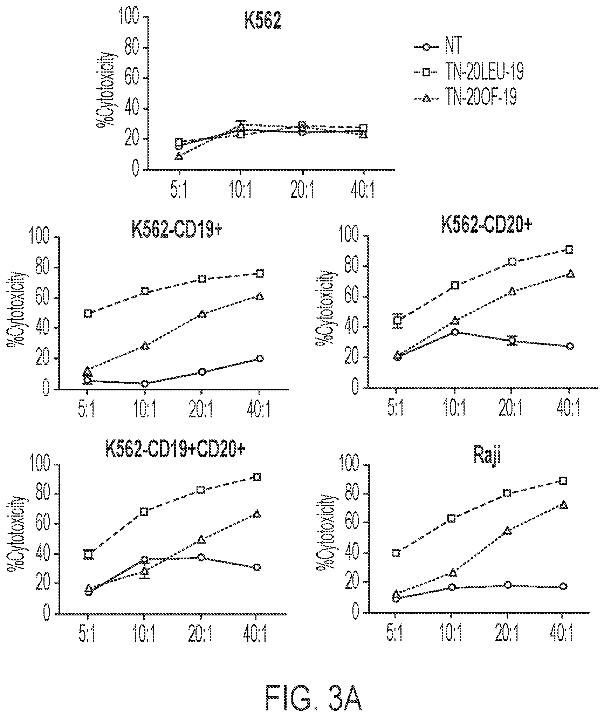

Figure 3A shows the detection of tumor-killing activity of CAR-T19/20s cells, mainly by detecting the secretion level of LDH in the supernatant after co-culture. Specifically, 1×10⁴ cells of CD19-positive K562-CD19+ tumor cell line, CD20-positive K562-CD20+ tumor cell line, CD19 and CD20 double positive K562-CD19+ CD20+ tumor cell line, RAJI or RAMOS tumor cell line that naturally expresses CD19 and CD20, or CD19 and CD20 double negative K562 tumor cell line were co-cultured with the corresponding T cells, respectively, in 100 µl of GT-551 medium for 8h with a ratio as shown in the figure. Then the secretion level of LDH was detected, and this figure shows the statistical analysis results of the percentages of LDH release in corresponding co-culture samples.

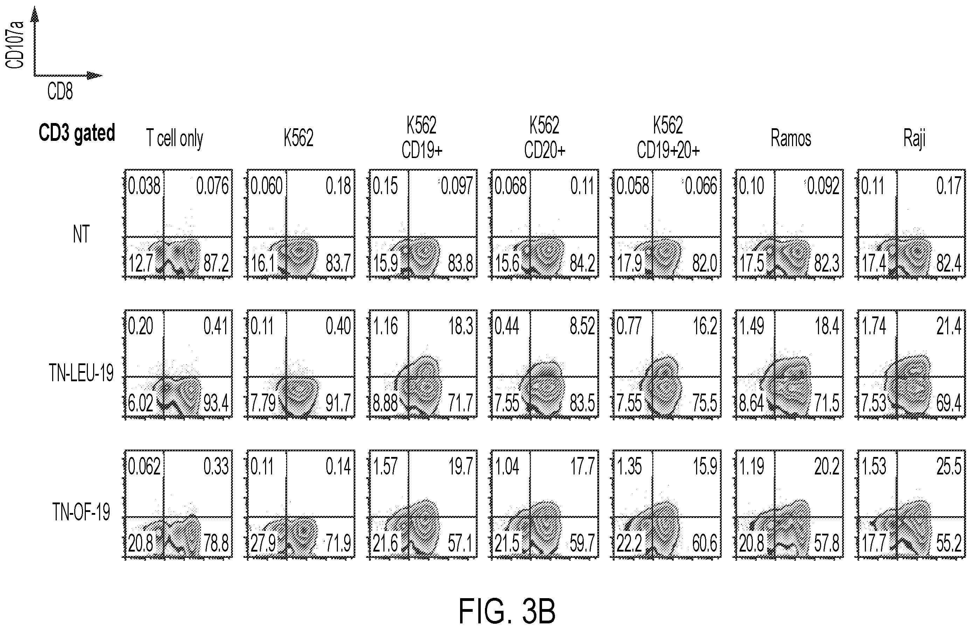

Figure 3B shows the detection of tumor-killing activity of CAR-T19/20s cells, mainly by detecting the expression level of CD107a on the surface of T cell membrane. Specifically, 1×10⁵ CAR-T19/20s cells were taken and cultured respectively with CD19-positive K562-CD19+ tumor cell line, CD20-positive K562-CD20+ tumor cell line, CD19 and CD20 double positive K562-CD19+CD20+ tumor cell line, RAJI or RAMOS tumor cell line that naturally expresses CD19 and CD20, and CD19 and CD20 double negative K562 tumor cell line, or without tumor cells, in 200 µl of GT-551 medium for 4h with a ratio of 1:2. Then the expression level of CD137 on the surface of T cell membrane was detected respectively.
Figure 4A shows the average body weight changes and average fluorescence intensity changes of mice injected with CAR-T19/20s cells within 21 days, which are recorded every 7 days.
Figure 4B shows the average fluorescence intensity of the three groups of mice.
Figure 4C shows *in vivo* imaging of mice injected with CAR-T19/20s cells on day 0 (D0), 7 (D7), 14 (D14), and 21 (D21) after injection.
Figure 5A shows that bi-specific TN-OF-19 CAR-T cells can inhibit or kill tumor cells better than CD19 specific CAR-T cells *in vivo.*
Figure 5B shows the IVIS imaging of fluorescence intensity in 4 CAR-T cell groups and NT group.
Figure 6A shows the structure of CD20scFv-CD19scFv rabFc.
Figure 6B shows the chimeric antibody bound to cells with CD19 or CD20 or both, but not to cells lacking both antigens, indicating that the bispecific binding domain required only one cognate antigen for binding and no new specific recognition sites were formed.
Figure 7 shows the structures of sixteen CD20 specific CARs with six different scFVs and different hinge/TM/signaling domains.
Figure 8A shows the results of IFNγ release assay to screen CAR-T20.1, CAR-T20.5, CAR-T20.6, CAR-T20.7, CAR-T20.8, CAR-T20.9 and CAR-T20.10, and among these CAR-Ts, only CAR-T20.9 (Leu16) and CAR-T20.10 (Leu16) showed higher positive IFNγ release.
Figure 8B shows the results of IFNγ release assay to screen CAR-T20.1, CAR-T20.9, CAR-T20.10, CAR-T20.11, CAR-T20.12, CAR-T20.13, CAR-T20.14, CAR-T20.15 and CAR-T20.16. Among these CAR-Ts, CAR-T20.10 (Leu16) and CAR-T20.14 (OF) showed higher positive IFNγ release.
Figure 8C shows the results of IFNγ release assay to screen CAR-T20.9, CAR-T20.12, CAR-T20.14, CAR-T20.17, CAR-T20.18, and CAR-T20.19. Among these CAR-Ts, CAR-T20.14 (OF) and CAR-T20.19 (OF) showed higher positive IFNγ release.
Figure 9A shows the results of CAR-T20.17 (Leu16 3^{rd} generation), CAR-T20.18 (Leu16 2^{nd} generation), CAR-T20.19 (OF 2^{nd} generation) cells tested for cytotoxicity by LDH release assay.
Figure 9B shows the *in vivo* inhibition of tumor growth in NSG mouse studies. The tumor cells injected in animals are Raji expressing luciferase.
Figure 10 shows CAR039 Phase I clinical study design and flow chart.
Figure 11 shows the summary of C-CAR039 clinical results.
Figure 12 shows examples of patient before and post C-CAR039 treatment.
Figure 13 shows C-CAR039 proliferation and expansion, as well as B cell depletion in patient's peripheral blood. The results showed that C-CAR008 cells expanded effectively after injection. Square: 1x10⁶ CAR-T cells/kg. Triangle: 2~2.5x10⁶ CAR-T cells/kg. Circle: 4~5.0x10⁶ CAR-T cells/kg. Solid symbols (square, triangle, circle): CAR-T DNA copy. Unfilled symbols (square, triangle, circle): CD19/CD20+ B cell level.
Figure 14 shows the C-CAR066-NHL study design.
Figure 15 shows an example of PET-CT of a patient before and after treated with C-CAR066.
Figures 16A-16D: Identification of the optimal antigen binding domain for anti-CD19/CD20 bispecific CAR. Figure 16A shows the structures of TN-OF-19 (C-CAR039) and TN-19-OF. Figure 16B shows that C-CAR039 with the tandem link of Ofatumumab scFv followed by the FMC63 scFv has superior anti-tumor activity. Figure 16C shows the structures of TN-OF-19 (C-CAR039), TN-OFR-19, TN-OF-19R and TN-OFR-19R. Figure 16D shows the superior anti-tumor activity and specificity link to the optimal structure of the antigen binding domain of C-CAR039.
Figure 17A shows the process of evaluating binding specificity of the antigen binding domain of C-CAR039. Figure 17B shows that CD19, CD20 and FCGR1A were identified with moderate binding at the concentrations of 5.0 µg/mL. No other significant interactions were identified. FCGR1 was reported to bind rabFc.
Figure 18 shows the Kaplan Meyer estimation of progression-free survival (PFS) for C-CAR039.
Figure 19 shows the pharmacokinetics (PK)/pharmacodynamics (PD) profile of C-CAR039. Low dose ("Low"): 1x10⁶ CAR-T cells/kg body weight; medium dose ("Mid"): 2x10⁶ - 2.5x10⁶ CAR-T cells/kg body weight; high dose ("High"): 4x10⁶ - 5.0x10⁶ CAR-T cells/kg body weight.
Figure 20 shows the PK/PD profile (CAR-T expansion and B cell depletion) in the peripheral blood of the relapsed patients.

### Detailed Description

After extensive and intensive studies, the inventors unexpectedly obtained a CAR-T cell that simultaneously targets CD19 and CD20. Specifically, the present invention provides a chimeric antigen receptor that simultaneously targets CD19 and CD20, which comprises a signal peptide, an anti-CD20 scFv, an anti-CD19 scFv, a hinge region, a transmembrane region, and an intracellular T cell signaling region. Moreover, the anti-CD20 scFv and anti-CD19 scFv were obtained through a large number of screenings, which were linked with peptide fragment with multiple repeat structure (G4S). The CAR-T cells of the present invention can recognize both CD19 and CD20 antigens at the same time, reducing the risk of immune escape caused by down-regulation or deletion of antigen expression during the treatment of single-target CAR-T cells. Compared to CAR-T cells targeting single antigens and other double target CAR-T cells (targeting CD19 and CD20), the CAR-T cells of the present invention that simultaneously recognize two targets have stronger killing ability against tumor cells, less cytotoxicity, lower side effects, wider treatment range, lower recurrence rate and better efficacy. The present invention has been completed on the basis of this.

### Terms

To make the disclosure easier to understand, some terms are firstly defined. As used in this application, unless expressly stated otherwise herein, each of the following terms shall have the meanings given below. Other definitions are set forth throughout the application.

The term "about" may refer to a value or composition within an acceptable error range for a particular value or composition as determined by those skilled in the art, which will depend in part on how the value or composition is measured or determined.

The term "administering" refers to the physical introduction of a product of the invention into a subject using any one of various methods and delivery systems known to those skilled in the art, including intravenous, intramuscular, subcutaneous, intraperitoneal, spinal or other parenteral administration, such as by injection or infusion.

The term "antibody" (Ab) may comprise, but is not limited to, an immunoglobulin that specifically binds an antigen and contains at least two heavy (H) chains and two light (L) chains linked by disulfide bonds, or an antigen binding parts thereof. Each H chain contains a heavy chain variable region (abbreviated herein as V_{H}) and a heavy chain constant region. The heavy chain constant region contains three constant domains, CH1, CH2, and CH3. Each light chain contains a light chain variable region (abbreviated herein as V_{L}) and a light chain constant region. The light chain constant region contains a constant domain C_{L}. The V_{H} and V_{L} regions can be further subdivided into hypervariable regions called complementarity determining regions (CDR), which are interspersed within more conservative regions called framework regions (FR). Each VH and VL contains three CDRs and four FRs, which are arranged from amino terminal to carboxy terminal in the following order: FR1, CDR1, FR2, CDR2, FR3, CDR3, and FR4. The variable regions of the heavy and light chains contain a binding domain that interacts with an antigen.

### CD20

Although the efficacy of anti-CD19 CAR-T is outstanding, many studies have shown that there are still many problems with CD19 chimeric antigen receptor (CAR) T cell therapy. There are still some patients with poor treatment results and easy to relapse. This includes the susceptibility of tumor cells to antigen escape.

In order to prevent the escape of CD19 CAR-T antigen, the inventors designed a combined bispecific CAR (i.e., BICAR) that targets both CD19 and CD20, so that when CD19 antigen escapes and is not expressed in tumor cells, CD20 can be recognized to clear tumor cells *in vivo.*

CD20 is expressed in most patients with B-cell acute lymphoblastic leukemia, including some CD19 negative patients after anti-CD19 CAR-T treatment. CD20 is a glycosylated protein, and is the first identified B cell membrane marker. CD20 is also known as B1, and encoded by the MS4A gene. CD20 molecule has four transmembrane hydrophobic regions, and its N-terminal and C-terminal are located on the cytoplasmic side, forming two closed loops outside the cell, and respectively called big loop and small loop. CD20 is specifically expressed in more than 95% of normal and cancerous B cells. These cells are in the pre-B cell stage and subsequent developmental stages, and CD20 stops expression until the cells differentiated into plasma cells. The present invention uses CD20 as another target for immunotherapy of B cell malignancies.

### Bispecific chimeric antigen receptor targeting CD19 and CD20

Cellular immunotherapy is an emerging and highly effective tumor treatment model, and is a new type of autoimmunology treatment for cancer. It is a method for *in vitro* culture and amplification of immune cells collected from a patient using biotechnology and biological agents, and then the cells are transfused back to the patient to stimulate and enhance the body's autoimmune function, thereby achieving the purpose of treating tumors. The skilled in the art have been working to develop new cellular immunotherapy to increase the efficiency and reduce the side effect.

The present invention proposes a rational and optimized single-chain design and system, that is, combined bispecific CAR, which can be effectively integrated into primary human T cells, and can simultaneously target CD19 and CD20 when the T cells are activated. The CAR-T cells of the invention are capable of recognizing two antigens (CD19 and CD20). The invention provides a very effective potential method for preventing antigen escape.

The present invention uses CAR that simultaneously targets CD19 and CD20. Compared with CARs that target a single antigen, the affinity is enhanced, the activity of T cells is increased, and the targets have an additive or synergistic effect. In addition, due to uneven expression levels of CD19 and CD20 in tumor cells, double target CAR-T therapy has a wider scope. The CAR-T that simultaneously targets CD19 and CD20 on the surface of tumor cells can reduce the possibility of antigen escape caused by down-regulation or deletion of single surface antigen.

Bispecificity means that the same CAR can specifically bind and immunorecognize two different antigens, and the CAR can generate an immune response by binding to any one of the antigens.

The CD19 and CD20 bispecific CAR of the present invention has a single structure and comprises anti-CD19 and anti-CD20 scFvs. Wherein, the CAR comprises a CD19 scFv and a CD20 scFv, and the amino acid sequences, sequencing and hinge of CD19 scFv and CD20 scFv are the main factors affecting its function.

Specifically, the chimeric antigen receptor (CAR) of the invention comprises an extracellular domain, a transmembrane domain, and an intracellular domain. The extracellular domain comprises a target-specific binding element (also known as an antigen binding domain). The intracellular domain comprises a co-stimulatory signaling region and a ζ chain. The co-stimulatory signaling region refers to a part of the intracellular domain that comprises a co-stimulatory molecule. The co-stimulatory molecule is a cell surface molecule required for efficient response of lymphocytes to antigens, rather than an antigen receptor or ligand thereof.

A linker can be incorporated between the extracellular domain and the transmembrane domain of the CAR, or between the cytoplasmic domain and the transmembrane domain of the CAR. As used herein, the term "linker" generally refers to any oligopeptide or polypeptide that plays a role of linking the transmembrane domain to the extracellular domain or the cytoplasmic domain in a polypeptide chain. The linker may comprise 0-300 amino acids, preferably 2-100 amino acids and most preferably 3-50 amino acids.

The extracellular domain of the CAR provided in the present invention comprises an antigen binding domain targeting CD19 and CD20. When the CAR of the present invention is expressed in T cell, antigen recognition can be performed based on antigen binding specificity. When the CAR binds to its associated antigen, it affects tumor cell, causing tumor cell to fail to grow, to death or to be affected otherwise, causing the patient's tumor burden to shrink or eliminate. The antigen binding domain is preferably fused to the intracellular domain from one or more of the co-stimulatory molecule and the ζ chain. Preferably, the antigen binding domain is fused with an intracellular domain of a combination of a 4-1BB signaling domain and a CD3ζ signaling domain.

As used herein, the "antigen binding domain" and "single-chain antibody fragment" refer to an Fab fragment, an Fab' fragment, an F (ab')₂ fragment, or a single Fv fragment that has antigen-binding activity. The Fv antibody contains the heavy chain variable region and the light chain variable region of the antibody, but has no constant region. The Fv antibody has the smallest antibody fragment with all antigen-binding sites. Generally, Fv antibodies also comprise a polypeptide linker between the V_{H} and V_{L} domains, and can form the structure required for antigen binding. The antigen binding domain is usually a scFv (single-chain variable fragment). The size of scFv is typically 1/6 of a complete antibody. The single-chain antibody is preferably an amino acid chain sequence encoded by a nucleotide chain. As a preferred embodiment of the present invention, the scFv comprises antibodies that specifically recognize CD19 and CD20.

As for the hinge region and the transmembrane region (transmembrane domain), the CAR can be designed to comprise a transmembrane domain fused to the extracellular domain of the CAR. In one embodiment, a transmembrane domain that is naturally associated with one of the domains in the CAR is used. In some embodiments, transmembrane domains may be selected or modified by amino acid substitutions to avoid binding such domains to the transmembrane domain of the same or different surface membrane proteins, thereby minimizing the interaction with other members of the receptor complexes.

The intracellular domain in the CAR of the invention comprises the signaling domain of 4-1BB and the signaling domain of CD3ζ.

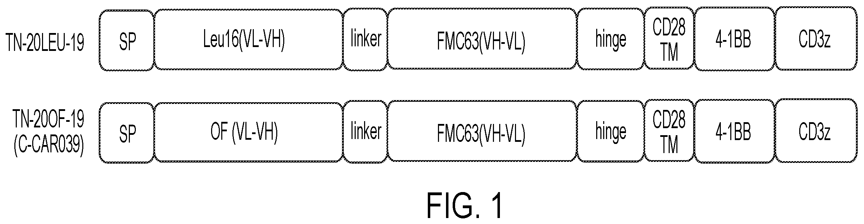

Preferably, the CAR structure of the present invention, in turn, comprises a signal peptide sequence (also known as leader sequence), an antigen recognition sequence (antigen-binding domain), a hinge region, a transmembrane region, a co-stimulatory factor signal region, and a CD3zeta signaling region (ζ chain portion). The order of connection is shown in figure 1.

A CAR described herein is TN-LEU-19. The antigen binding domain targeting CD20

| **Leu16 Region** | **Sequence** | **Residues** | **Length** |
|---|---|---|---|
| HFR1 | EVQLQQSGAELVKPGASVKMSCKASGYTFT (SEQ ID NO: 46) | 1 - 30 | 30 |
| V_{H}-CDR1 | SYNMH (SEQ ID NO: 47) | 31 - 35 | 5 |
| HFR2 | WVKQTPGQGLEWIG (SEQ ID NO: 48) | 36 - 49 | 14 |
| V_{H}-CDR2 | AIYPGNGDTSYNQKFKG (SEQ ID NO: 49) | 50 - 66 | 17 |
| HFR3 | KATLTADKSSSTAYMQLSSLTSEDSADYYCAR (SEQ ID NO: 50) | 67 - 98 | 32 |
| V_{H}-CDR3 | SNYYGSSYWFFDV (SEQ ID NO: 51) | 99 - 111 | 13 |
| HFR4 | WGAGTTVTVSS (SEQ ID NO: 52) | 112 - 122 | 11 |
| Leu16 V_{H} | | | 122 |

comprises a heavy chain sequence (SEQ ID NO: 1) and a light chain (VL) sequence (SEQ ID NO: 2) of the single-chain variable region derived from Leu16 antibody.

Heavy chain sequence of single-chain variable region (V_{H}) derived from Leu16 antibody: 
EVQLQQSGAELVKPGASVKMSCKASGYTFTSYNMHWVKQTPGQGLEWIGAIYP
GNGDTSYNQKFKGKATLTADKSSSTAYMQLSSLTSEDSADYYCARSNYYGSSYWFFD
VWGAGTTVTVSS (SEQ ID NO: 1)
LEU-VH-CDR1: SEQ ID NO: 1, position 31-35;
LEU -VH-CDR2: SEQ ID NO: 1, position 50-66;
LEU -VH-CDR3: SEQ ID NO: 1, position 99-111.

Light chain sequence of single-chain variable region (V_{L}) derived from Leu16 antibody: 
DIVLTQSPAILSASPGEKVTMTCRASSSVNYMDWYQKKPGSSPKPWIYATSNLAS
GVPARFSGSGSGTSYSLTISRVEAEDAATYYCQQWSFNPPTFGGGTKLEIK (SEQ ID NO:
2)
LEU-VL-CDR1: SEQ ID NO: 2, position 24-33;
LEU -VL-CDR2: SEQ ID NO: 2, position 49-55;
LEU -VL-CDR3: SEQ ID NO: 2, position 88-96.

| **Leu16 Region** | **Sequence** | **Residues** | **Length** |
|---|---|---|---|
| LFR1 | DIVLTQSPAILSASPGEKVTMTC (SEQ ID NO: 53) | 1 - 23 | 23 |
| V_{L}-CDR1 | RASSSVNYMD (SEQ ID NO: 54) | 24 - 33 | 10 |
| LFR2 | WYQKKPGSSPKPWIY (SEQ ID NO: 55) | 34 - 48 | 15 |
| V_{L}-CDR2 | ATSNLAS (SEQ ID NO: 56) | 49 - 55 | 7 |
| LFR3 | GVPARFSGSGSGTSYSLTISRVEAEDAATYYC (SEQ ID NO: 57) | 56 - 87 | 32 |
| V_{L}-CDR3 | QQWSFNPPT (SEQ ID NO: 58) | 88 - 96 | 9 |
| LFR4 | FGGGTKLEIK (SEQ ID NO: 59) | 97 - 106 | 10 |
| Leu16 V_{L} | | | 106 |

The CAR of the present invention is TN-OF-19. The antigen binding domain targeting CD20 comprises a heavy chain sequence (SEQ ID NO: 3) and a light chain sequence (SEQ ID NO: 4) of the single-chain variable region derived from Ofatumumab antibody.

Heavy chain sequence of single-chain variable region (VH) derived from Ofatumumab antibody:

EVQLVESGGGLVQPGRSLRLSCAASGFTFNDYAMHWVRQAPGKGLEWVSTISW
NSGSIGYADSVKGRFTISRDNAKKSLYLQMNSLRAEDTALYYCAKDIQYGNYYYGMD
VWGQGTTVTVSS (SEQ ID NO: 3)

OF-VH-CDR1: SEQ ID NO: 3, position 30-35. The sequence of OF-VH-CDR1 is: NDYAMH (SEQ ID NO: 40).

OF-VH-CDR2: SEQ ID NO: 3, position 50-66. The sequence of OF-VH-CDR2 is: TISWNSGSIGYADSVKG (SEQ ID NO: 41).

OF-VH-CDR3: SEQ ID NO: 3, position 99-111. The sequence of OF-VH-CDR3 is: DIQYGNYYYGMDV (SEQ ID NO: 42).

Light chain sequence of single-chain variable region (VL) derived from Ofatumumab antibody:

EIVLTQSPATLSLSPGERATLSCRASQSVSSYLAWYQQKPGQAPRLLIYDASNRAT
GIPARFSGSGSGTDFTLTISSLEPEDFAVYYCQQRSNWPITFGQGTRLEIK (SEQ ID NO: 4)

OF-VL-CDR1: SEQ ID NO: 4, position 24-34. The sequence of OF-VL-CDR1 is: RASQSVSSYLA (SEQ ID NO: 43).

OF-VL-CDR2: SEQ ID NO: 4, position 50-56. The sequence of OF-VL-CDR2 is: DASNRAT (SEQ ID NO: 44).

OF-VL-CDR3: SEQ ID NO: 4, position 89-97. The sequence of OF-VL-CDR3 is: QQRSNWPIT (SEQ ID NO: 45).

In another preferred embodiment, the antigen-binding domain targeting CD19 in the CAR of the present invention comprises a light chain (V_{L}) sequence (SEQ ID NO: 5) and a heavy chain (V_{H}) sequence (SEQ ID NO: 6) of the single-chain variable region derived from FMC63 antibody.

Amino acid sequence of the light chain of single-chain variable region (V_{L}) derived from FMC63 antibody:

DIQMTQTTSSLSASLGDRVTISCRASQDISKYLNWYQQKPDGTVKLLIYHTSRLHSGVP
SRFSGSGSGTDYSLTISNLEQEDIATYFCQQGNTLPYTFGGGTKLEIT(SEQ ID NO: 5)

FMC63-VL-CDR1: SEQ ID NO: 5, position 24-34. The sequence of FMC63-VL-CDR1 is: RASQDISKYLN (SEQ ID NO: 63).

FMC63-VL-CDR2: SEQ ID NO: 5, position 50-56. The sequence of FMC63-VL-CDR2 is: HTSRLHS (SEQ ID NO: 64).

FMC63-VL-CDR3: SEQ ID NO: 5, position 89-97. The sequence of FMC63-VL-CDR3 is: QQGNTLPYT (SEQ ID NO: 39).

Nucleotide sequence of the light chain of single-chain variable region (V_{L}) derived from FMC63 antibody:

gacatccaga tgacacagac tacatcctcc ctgtctgcct ctctgggaga cagagtcacc 60
atcagttgca gggcaagtca ggacattagt aaatatttaa attggtatca gcagaaacca 120
gatggaactg ttaaactcct gatctaccat acatcaagat tacactcagg agtcccatca 180
aggttcagtg gcagtgggtc tggaacagat tattctctca ccattagcaa cctggagcaa 240
gaagatattg ccacttactt ttgccaacag ggtaatacgc ttccgtacac gttcggaggg 300
gggaccaagc tggagatcac a 321 (SEQ ID NO: 21)

Amino acid sequence of the heavy chain of single-chain variable region (V_{H}) derived from FMC63 antibody:

EVKLQESGPGLVAPSQSLSVTCTVSGVSLPDYGVSWIRQPPRKGLEWLGVIWGSE
TTYYNSALKSRLTIIKDNSKSQVFLKMNSLQTDDTAIYYCAKHYYYGGSYAMDYWGQ
GTSVTVSS (SEQ ID NO: 6)

FMC63-VH-CDR1: SEQ ID NO: 6, position 31-35. The sequence of FMC63-VH-CDR1 is: DYGVS (SEQ ID NO: 60).

FMC63-VH-CDR2: SEQ ID NO: 6, position 50-65. The sequence of FMC63-VH-CDR2 is: VIWGSETTYYNSALKS (SEQ ID NO: 61).

FMC63-VH-CDR3: SEQ ID NO: 6, position 98-109. The sequence of FMC63-VH-CDR3 is: HYYYGGSYAMDY (SEQ ID NO: 62).

Nucleotide sequence of the heavy chain of single-chain variable region (VH) derived from FMC63 antibody:

gaggtgaaac tgcaggagtc aggacctggc ctggtggcgc cctcacagag cctgtccgtc 60

acatgcactg tctcaggggt ctcattaccc gactatggtg taagctggat tcgccagcct 120

ccacgaaagg gtctggagtg gctgggagta atatggggta gtgaaaccac atactataat 180

tcagctctca aatccagact gaccatcatc aaggacaact ccaagagcca agttttctta 240

aaaatgaaca gtctgcaaac tgatgacaca gccatttact actgtgccaa acattattac 300

tacggtggta gctatgctat ggactactgg ggccaaggaa cctcagtcac cgtctcctca 360

(SEQ ID NO: 22)

Specifically, the sequences of other elements in the CAR of the present invention are as follows:
The leader sequence is the leader sequence of CD8 antigen:
MALPVTALLLPLALLLHAARP (SEQ ID NO: 8)

The linker sequences (i.e., flexible linker I) between the heavy chain and light chain of the single-chain variable region are:
Amino acid sequence of the linker between V_{L} and V_{H} of CD20 scfv:
GSTSGGGSGGGSGGGGSS (SEQ ID NO: 19)

Amino acid sequence of the linker between V_{H} and V_{L} of CD19 scFv:
GSTSGSGKPGSGEGSTKG (SEQ ID NO: 20)

The hinge region is selected from the sequence of a short form of IgG4 Hinge:
ESKYGPPCPPCP (SEQ ID NO: 9)

The transmembrane region is the transmembrane region sequence of CD8 (CD8TM) or CD28 (CD28TM) antigen:
CD8TM: IYIWAPLAGTCGVLLLSLVITLYC (SEQ ID NO: 10)
CD28TM: MFWVLVVVGGVLACYSLLVTVAFIIFWV (SEQ ID NO: 11)

The co-stimulatory factor signal region is derived from the sequence of 4-1BB or CD28 cytoplasmic signaling motif:
4-1BB: KRGRKKLLYIFKQPFMRPVQTTQEEDGCSCRFPEEEEGGCEL (SEQ ID NO: 12)
CD28: RSKRSRLLHSDYMNMTPRRPGPTRKHYQPYAPPRDFAAYRS (SEQ ID NO: 13)

The signaling region of CD3ζ is derived from the sequence of immunoreceptor tyrosine-based activation motif (ITAM) of CD3ζ in the TCR complex:

RVKFSRSADAPAYQQGQNQLYNELNLGRREEYDVLDKRRGRDPEMGGKPRRKNP
QEGLYNELQKDKMAEAYSEIGMKGERRRGKGHDGLYQGLSTATKDTYDALHMQALPP
R (SEQ ID NO: 14)

In a preferred embodiment, the complete nucleic acid sequences and amino acid sequences of the two CARs described herein are as follows

The complete nucleic acid sequence of TN-OF-19 of the invention is as follows:

ATGGCCTTACCAGTGACCGCCTTGCTCCTGCCGCTGGCCTTGCTGCTCCACGCC
GCCAGGCCGGAAATTGTGTTGACACAGTCTCCAGCCACCCTGTCTTTGTCTCCAGGG
GAAAGAGCCACCCTCTCCTGCAGGGCCAGTCAGAGTGTTAGCAGCTACTTAGCCTG
GTACCAACAGAAACCTGGCCAGGCTCCCAGGCTCCTCATCTATGATGCATCCAACAG
GGCCACTGGCATCCCAGCCAGGTTCAGTGGCAGTGGGTCTGGGACAGACTTCACTCT
CACCATCAGCAGCCTAGAGCCTGAAGATTTTGCAGTTTATTACTGTCAGCAGCGTAG
CAACTGGCCGATCACCTTCGGCCAAGGGACACGACTGGAGATTAAAGGCAGTACTA
GCGGTGGTGGCTCCGGGGGCGGTTCCGGTGGGGGCGGCAGCAGCGAAGTGCAGCTG
GTGGAGTCTGGGGGAGGCTTGGTACAGCCTGGCAGGTCCCTGAGACTCTCCTGTGCA
GCCTCTGGATTCACCTTTAATGATTATGCCATGCACTGGGTCCGGCAAGCTCCAGGG
AAGGGCCTGGAGTGGGTCTCAACTATTAGTTGGAATAGTGGTTCCATAGGCTATGCG
GACTCTGTGAAGGGCCGATTCACCATCTCCAGAGACAACGCCAAGAAGTCCCTGTA
TCTGCAAATGAACAGTCTGAGAGCTGAGGACACGGCCTTGTATTACTGTGCAAAAG
ATATACAGTACGGCAACTACTACTACGGTATGGACGTCTGGGGCCAAGGGACCACG
GTCACCGTCTCCTCAGGAGGTGGTGGATCCGAGGTGAAGCTGCAGGAAAGCGGCCC
TGGCCTGGTGGCCCCCAGCCAGAGCCTGAGCGTGACCTGCACCGTGAGCGGCGTGA
GCCTGCCCGACTACGGCGTGAGCTGGATCCGGCAGCCCCCCAGGAAGGGCCTGGAA
TGGCTGGGCGTGATCTGGGGCAGCGAGACCACCTACTACAACAGCGCCCTGAAGAG
CCGGCTGACCATCATCAAGGACAACAGCAAGAGCCAGGTGTTCCTGAAGATGAACA
GCCTGCAGACCGACGACACCGCCATCTACTACTGCGCCAAGCACTACTACTACGGC

GGCAGCTACGCCATGGACTACTGGGGCCAGGGCACCAGCGTGACCGTGAGCAGCGG
CAGCACCTCCGGCAGCGGCAAGCCTGGCAGCGGCGAGGGCAGCACCAAGGGCGAC
ATCCAGATGACCCAGACCACCTCCAGCCTGAGCGCCAGCCTGGGCGACCGGGTGAC
CATCAGCTGCCGGGCCAGCCAGGACATCAGCAAGTACCTGAACTGGTATCAGCAGA
AGCCCGACGGCACCGTCAAGCTGCTGATCTACCACACCAGCCGGCTGCACAGCGGC
GTGCCCAGCCGGTTTAGCGGCAGCGGCTCCGGCACCGACTACAGCCTGACCATCTCC
AACCTGGAACAGGAAGATATCGCCACCTACTTTTGCCAGCAGGGCAACACACTGCC
CTACACCTTTGGCGGCGGAACAAAGCTGGAAATCACCGAGAGCAAGTACGGACCGC
CCTGCCCCCCTTGCCCTATGTTCTGGGTGCTGGTGGTCGGAGGCGTGCTGGCCT
GCTACAGCCTGCTGGTCACCGTGGCCTTCATCATCTTTTGGGTGAAACGGGGCAGAA
AGAAACTCCTGTATATATTCAAACAACCATTTATGAGACCAGTACAAACTACTCAAG
AGGAAGATGGCTGTAGCTGCCGATTTCCAGAAGAAGAAGGAGGATGTGAACTG
CGGGTGAAGTTCAGCAGAAGCGCCGACGCCCCTGCCTACCAGCAGGGCCAGAATCA
GCTGTACAACGAGCTGAACCTGGGCAGAAGGGAAGAGTACGACGTCCTGGATAAGC
GGAGAGGCCGGGACCCTGAGATGGGCGGCAAGCCTCGGCGGAAGAACCCCCAGGA
AGGCCTGTATAACGAACTGCAGAAAGACAAGATGGCCGAGGCCTACAGCGAGATCG
GCATGAAGGGCGAGCGGAGGCGGGGCAAGGGCCACGACGGCCTGTATCAGGGCCT
GTCCACCGCCACCAAGGATACCTACGACGCCCTGCACATGCAGGCCCTGCCCCCAA
GG (SEQ ID NO:18)

The complete amino acid sequence of TN-OF-19 is as follows:

MALPVTALLLPLALLLHAARPEIVLTQSPATLSLSPGERATLSCRASQSVSSYLAW
YQQKPGQAPRLLIYDASNRATGIPARFSGSGSGTDFTLTISSLEPEDFAVYYCQQRSNWPI
TFGQGTRLEIKGSTSGGGSGGGSGGGGSSEVQLVESGGGLVQPGRSLRLSCAASGFTFN
DYAMHWVRQAPGKGLEWVSTISWNSGSIGYADSVKGRFTISRDNAKKSLYLQMNSLR
AEDTALYYCAKDIQYGNYYYGMDVWGQGTTVTVSSGGGGSEVKLQESGPGLVAPSQS
LSVTCTVSGVSLPDYGVSWIRQPPRKGLEWLGVIWGSETTYYNSALKSRLTIIKDNSKSQ
VFLKMNSLQTDDTAIYYCAKHYYYGGSYAMDYWGQGTSVTVSSGSTSGSGKPGSGEG
STKGDIQMTQTTSSLSASLGDRVTISCRASQDISKYLNWYQQKPDGTVKLLIYHTSRLHS
GVPSRFSGSGSGTDYSLTISNLEQEDIATYFCQQGNTLPYTFGGGTKLEITESKYGPPCPP
CPMFWVLVVVGGVLACYSLLVTVAFIIFWVKRGRKKLLYIFKQPFMRPVQTTQEEDGC
SCRFPEEEEGGCELRVKFSRSADAPAYQQGQNQLYNELNLGRREEYDVLDKRRGRDPE

MGGKPRRKNPQEGLYNELQKDKMAEAYSEIGMKGERRRGKGHDGLYQGLSTATKDTY
DALHMQALPPR (SEQ ID NO: 16)

The complete nucleic acid sequence of TN-LEU-19 (reference) is as follows:

ATGGAGACAGACACACTCCTGCTATGGGTGCTGCTGCTCTGGGTTCCAGGTTCC
ACAGGTGACATTGTGCTGACCCAATCTCCAGCTATCCTGTCTGCATCTCCAGGGGAG
AAGGTCACAATGACTTGCAGGGCCAGCTCAAGTGTAAATTACATGGACTGGTACCA
GAAGAAGCCAGGATCCTCCCCCAAACCCTGGATTTATGCCACATCCAACCTGGCTTC
TGGAGTCCCTGCTCGCTTCAGTGGCAGTGGGTCTGGGACCTCTTACTCTCTCACAATC
AGCAGAGTGGAGGCTGAAGATGCTGCCACTTATTACTGCCAGCAGTGGAGTTTTAAT
CCACCCACGTTCGGAGGGGGGACCAAGCTGGAAATAAAAGGCAGTACTAGCGGTGG
TGGCTCCGGGGGCGGTTCCGGTGGGGGCGGCAGCAGCGAGGTGCAGCTGCAGCAGT
CTGGGGCTGAGCTGGTGAAGCCTGGGGCCTCAGTGAAGATGTCCTGCAAGGCTTCTG
GCTACACATTTACCAGTTACAATATGCACTGGGTAAAGCAGACACCTGGACAGGGC
CTGGAATGGATTGGAGCTATTTATCCAGGAAATGGTGATACTTCCTACAATCAGAAG
TTCAAAGGCAAGGCCACATTGACTGCAGACAAATCCTCCAGCACAGCCTACATGCA
GCTCAGCAGCCTGACATCTGAGGACTCTGCGGACTATTACTGTGCAAGATCTAATTA
TTACGGTAGTAGCTACTGGTTCTTCGATGTCTGGGGCGCAGGGACCACGGTCACCGT
CTCCTCAGGAGGTGGTGGATCCGAGGTGAAGCTGCAGGAAAGCGGCCCTGGCCTGG
TGGCCCCCAGCCAGAGCCTGAGCGTGACCTGCACCGTGAGCGGCGTGAGCCTGCCC
GACTACGGCGTGAGCTGGATCCGGCAGCCCCCCAGGAAGGGCCTGGAATGGCTGGG
CGTGATCTGGGGCAGCGAGACCACCTACTACAACAGCGCCCTGAAGAGCCGGCTGA
CCATCATCAAGGACAACAGCAAGAGCCAGGTGTTCCTGAAGATGAACAGCCTGCAG
ACCGACGACACCGCCATCTACTACTGCGCCAAGCACTACTACTACGGCGGCAGCTA
CGCCATGGACTACTGGGGCCAGGGCACCAGCGTGACCGTGAGCAGCGGCAGCACCT
CCGGCAGCGGCAAGCCTGGCAGCGGCGAGGGCAGCACCAAGGGCGACATCCAGAT
GACCCAGACCACCTCCAGCCTGAGCGCCAGCCTGGGCGACCGGGTGACCATCAGCT
GCCGGGCCAGCCAGGACATCAGCAAGTACCTGAACTGGTATCAGCAGAAGCCCGAC
GGCACCGTCAAGCTGCTGATCTACCACACCAGCCGGCTGCACAGCGGCGTGCCCAG
CCGGTTTAGCGGCAGCGGCTCCGGCACCGACTACAGCCTGACCATCTCCAACCTGGA
ACAGGAAGATATCGCCACCTACTTTTGCCAGCAGGGCAACACACTGCCCTACACCTT

TGGCGGCGGAACAAAGCTGGAAATCACCGAGAGCAAGTACGGACCGCCCTGCCCCC
CTTGCCCTATGTTCTGGGTGCTGGTGGTCGGAGGCGTGCTGGCCTGCTACAGCC
TGCTGGTCACCGTGGCCTTCATCATCTTTTGGGTGAAACGGGGCAGAAAGAAACTCC
TGTATATATTCAAACAACCATTTATGAGACCAGTACAAACTACTCAAGAGGAAGAT
GGCTGTAGCTGCCGATTTCCAGAAGAAGAAGAAGGAGGATGTGAACTGCGGGTGAA
GTTCAGCAGAAGCGCCGACGCCCCTGCCTACCAGCAGGGCCAGAATCAGCTGTACA
ACGAGCTGAACCTGGGCAGAAGGGAAGAGTACGACGTCCTGGATAAGCGGAGAGG
CCGGGACCCTGAGATGGGCGGCAAGCCTCGGCGGAAGAACCCCCAGGAAGGCCTGT
ATAACGAACTGCAGAAAGACAAGATGGCCGAGGCCTACAGCGAGATCGGCATGAA
GGGCGAGCGGAGGCGGGGCAAGGGCCACGACGGCCTGTATCAGGGCCTGTCCACCG
CCACCAAGGATACCTACGACGCCCTGCACATGCAGGCCCTGCCCCCAAGG(SEQ ID
NO:17)

The complete amino acid sequence of TN-LEU-19 is as follows:

METETDTLLLWVLLLWVPGSTGDIVLTQSPAILSASPGEKVTMTCRASSSVNYMDWYQK
KPGSSPKPWIYATSNLASGVPARFSGSGSGTSYSLTISRVEAEDAATYYCQQWSFNPPTFGGGT
KLEIKGSTSGGGSGGGSGGGGSSEVQLQQSGAELVKPGASVKMSCKASGYTFTSYNMH
WVKQTPGQGLEWIGAIYPGNGDTSYNQKFKGKATLTADKSSSTAYMQLSSLTSEDSAD
YYCARSNYYGSSYWFFDVWGAGTTVTVSSGGGGSEVKLQESGPGLVAPSQSLSVTCTV
SGVSLPDYGVSWIRQPPRKGLEWLGVIWGSETTYYNSALKSRLTIIKDNSKSQVFLKMN
SLQTDDTAIYYCAKHYYYGGSYAMDYWGQGTSVTVSSGSTSGSGKPGSGEGSTKGDIQ
MTQTTSSLSASLGDRVTISCRASQDISKYLNWYQQKPDGTVKLLIYHTSRLHSGVPSRFSGSGS
GTDYSLTISNLEQEDIATYFCQQGNTLPYTFGGGTKLEITESKYGPPCPPCPMFWVLVVVGG
VLACYSLLVTVAFIIFWVKRGRKKLLYIFKQPFMRPVQTTQEEDGCSCRFPEEEEGGCEL
RVKFSRSADAPAYQQGQNQLYNELNLGRREEYDVLDKRRGRDPEMGGKPRRKNPQEG
LYNELQKDKMAEAYSEIGMKGERRRGKGHDGLYQGLSTATKDTYDALHMQALPPR(SE
Q ID NO:15)

The design of the BICAR of the present invention has the following advantages:
First, CD19 and CD20 are expressed in most malignant B-cell tumors. Secondly, in general, when expanding CAR structure to increase T cell recognition ability, problems such as increased adverse targeting, increased cytotoxicity, and increased side effects are often encountered. However, this is not the case for CD19 and CD20 because both of them are only expressed in B cells with the same tumor toxicity curve. Finally, the expression of CD19 and CD20 in B cells can promote the survival of B cells. And the loss of both antigens during treatment is a very low probability event. Therefore, targeting CD19 and CD20 is expected to provide effective prevention of antigen escape of malignant B cells.

Compared with the single CAR of CD19 or CD20, BICAR has the following advantages:
First, compared with expressing two independent CARs, when expressing BICAR in a single T cell, the DNA footprint is significantly reduced (the DNA length is reduced by 40%). The size and the length of structure can significantly affect the packaging and transduction efficiency of the viral vector, thus directly affecting the clinical efficacy. Secondly, compared to the mixture of two different single CARs, BICAR can significantly reduce the cost of treatment (BICAR is completely compatible with the current T cell production process without adding additional burden.) and increase the clinical cure rate. Finally, CD19 and CD20 have been verified in a large number of clinical studies and are relatively safe.

In the present invention, we constructed two types of chimeric antigen receptor structures (TN-LEU-19, TH-OF-19) targeting CD19 and CD20 based on the sequence of CD19 mouse-derived monoclonal antibody FMC63 and the sequences of CD20 mouse-derived monoclonal antibody leu-16 and Ofatumumab. We completed the analysis and identification of the expression levels, *in vitro* activation capacity, and tumor cell killing efficacy of these two chimeric antigen receptors in primary T cells. Finally, it was found that the T-cells modified with TN-LEU-19 or TH-OF-19 chimeric antigen receptors have a strong ability to kill *in vitro* and to clear malignant tumors carrying CD19 and CD20 positive antigens *in vivo,* and Ofatumumab is better than leu16. This provides a new effective method and preparation for the clinical application of CAR-T in the treatment of CD19 and CD20-positive leukemias and lymphomas.

The present invention designed and optimized single-specific and double-specific CARs. These CARs have a powerful killing ability against B-cell malignancies expressing CD19 or CD20. BICAR allows a single T-cell product to target two clinically validated antigens associated with B-cell leukemia and lymphoma, ultimately reducing the risk of tumor recurrence due to the loss or escape of a single antigen. The invention can be further used in the design of new BICAR, thus increasing the antigen's applicability and increasing the efficacy of T cell therapy for cancer.

### Chimeric antigen receptor T cell (CAR-T cell)

As used herein, the terms "CAR-T cell", "CAR-T", "CART", "CAR-T cell of the present invention" all refer to the CAR-T cell that targets both CD19 and CD20 of the forth aspect of the invention. Specifically, the CAR structure of the CAR-T cells comprises an anti-CD19 scFv, an anti-CD20 scFv, a hinge region, a transmembrane region, and an intracellular T cell signaling region in turn, wherein the anti-CD20 scFv and anti-CD19 scFv are linked with a peptide having multiple repeating structures (G4S). Compared with CAR-T targeting a single antigen, the CAR-T cell that simultaneously recognizes two targets are more lethal and have a wider range of treatment.

### Vector

The nucleic acid sequences coding for the desired molecules can be obtained using recombinant methods known in the art, such as, for example by screening libraries from cells expressing the gene, by deriving the gene from a vector known to comprise the same, or by isolating directly from cells and tissues containing the same, using standard techniques. Alternatively, the gene of interest can be produced synthetically.

The present invention also provides vectors in which the expression cassette of the present invention is inserted. Vectors derived from retroviruses such as the lentivirus are suitable tools to achieve long-term gene transfer since they allow long-term, stable integration of a transgene and its propagation in daughter cells. Lentiviral vectors have the advantage over vectors derived from onco-retroviruses such as murine leukemia viruses in that they can transduce non-proliferating cells, such as hepatocytes. They also have the advantage of low immunogenicity.

In brief summary, the expression cassette or nucleic acid sequence of the invention is typically and operably linked to a promoter, and incorporated into an expression vector. The vectors can be suitable for replication and integration in eukaryotes. Typical cloning vectors contain transcription and translation terminators, initiation sequences, and promoters useful for regulation of the expression of the desired nucleic acid sequence.

The expression constructs of the present invention may also be used for nucleic acid immune and gene therapy, using standard gene delivery protocols. Methods for gene delivery are known in the art. See, e.g., U.S, Pat. Nos. 5,399,346, 5,580,859, 5,589,466. In another embodiment, the invention provides a gene therapy vector.

The nucleic acid can be cloned into a number of types of vectors. For example, the nucleic acid can be cloned into a vector including, but not limited to a plasmid, a phagemid, a phage derivative, an animal virus, and a cosmid. Vectors of particular interest comprise expression vectors, replication vectors, probe generation vectors, and sequencing vectors.

Further, the expression vector may be provided to a cell in the form of a viral vector. Viral vector technology is well known in the art and is described, for example, in Sambrook et al, (2001, Molecular Cloning: A Laboratory Manual, Cold Spring Harbor Laboratory, New York), and in other virology and molecular biology manuals. Viruses, which are useful as vectors comprise, but are not limited to, retroviruses, adenoviruses, adeno-associated viruses, herpes viruses, and lentiviruses. In general, a suitable vector contains an origin of replication functional in at least one organism, a promoter sequence, convenient restriction endonuclease sites, and one or more selectable markers, (e.g., WO 01/96584; WO 01/29058; and U.S, Pat. No. 6,326, 193).

A number of viral based systems have been developed for gene transfer into mammalian cells. For example, retroviruses provide a convenient platform for gene delivery systems. A selected gene can be inserted into a vector and packaged in retroviral particles using techniques known in the art. The recombinant virus can then be isolated and delivered to cells of the subject either *in vivo* or *ex vivo.* A number of retroviral systems are known in the art. In some embodiments, adenovirus vectors are used. A number of adenovirus vectors are known in the art. In one embodiment, lentivirus vectors are used.

Additional promoter elements, e.g., enhancers, regulate the frequency of transcriptional initiation. Typically, these are located in the region 30-1 10 bp upstream of the start site, although a number of promoters have recently been shown to contain functional elements downstream of the start site as well. The spacing between promoter elements frequently is flexible, so that promoter function is preserved when elements are inverted or moved relative to one another,. In the thymidine kinase (tk) promoter, the spacing between promoter elements can be increased to 50 bp apart before activity begins to decline. Depending on the promoter, it appears that individual elements can function either cooperatively or independently to activate transcription.

One example of a suitable promoter is the immediate early cytomegalovirus (CMV) promoter sequence. This promoter sequence is a strong constitutive promoter sequence capable of driving high levels of expression of any polynucleotide sequence operatively linked thereto. Another example of a suitable promoter is Elongation Growth Factor-1α (EF- 1α). However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the hemoglobin promoter, and the creatine kinase promoter. Further, the invention should not be limited to the use of constitutive promoters, inducible promoters are also contemplated as part of the invention. The use of an inducible promoter provides a molecular switch capable of turning on expression of the polynucleotide sequence which it is operatively linked when such expression is desired, or turning off the expression when expression is not desired. Examples of inducible promoters comprise, but are not limited to a metallothionein promoter, a glucocorticoid promoter, a progesterone promoter, and a tetracycline promoter.

In order to assess the expression of a CAR polypeptide or portions thereof, the expression vector to be introduced into a ceil can also contain either a selectable marker gene or a reporter gene or both to facilitate identification and selection of expressing cells from the population of cells sought to be transfected or infected through viral vectors. In other aspects, the selectable marker may be carried on a separate piece of DNA and used in a co- transfection procedure. Both selectable markers and reporter genes may be flanked with appropriate regulatory sequences to enable expression in the host cells. Useful selectable markers comprise, for example, antibiotic-resistance genes, such as neo and the like.

Reporter genes are used for identifying potentially transfected cells and for evaluating the functionality of regulatory sequences. In general, a reporter gene is a gene that is not present in or expressed by the recipient organism or tissue and that encodes a polypeptide whose expression is manifested by some easily detectable property, e.g., enzymatic activity. Expression of the reporter gene is assayed at a suitable time after the DNA has been introduced into the recipient cells. Suitable reporter genes may comprise genes encoding luciferase, beta-galactosidase, chloramphenicol acetyl transferase, secreted alkaline phosphatase, or the green fluorescent protein gene (e.g., Ui-Tei et al., 2000 FEBS Letters 479: 79-82). Suitable expression systems are well known and may be prepared using known techniques or obtained commercially. In general, the construct with the minimal 5' flanking region showing the highest level of expression of reporter gene is identified as the promoter. Such promoter regions may be linked to a reporter gene and used to evaluate agents for the ability to modulate promoter- driven transcription.

Methods of introducing and expressing genes into a cell are known in the art. In the context of an expression vector, the vector can be readily introduced into a host cell, e.g., mammalian, bacterial, yeast, or insect cell by any method in the art. For example, the expression vector can be transferred into a host cell by physical, chemical, or biological means.

Physical methods for introducing a polynucleotide into a host cell comprise calcium phosphate precipitation, lipofection, particle bombardment, microinjection, electroporation, and the like. Methods for producing cells comprising vectors and/or exogenous nucleic acids are well-known in the art. See, for example, Sambrook et al. (2001, Molecular Cloning: A Laboratory Manual, Cold Spring Harbor Laboratory, New York). A preferred method for the introduction of a polynucleotide into a host cell is calcium phosphate transfection.

Biological methods for introducing a polynucleotide of interest into a host cell comprise the use of DNA and RNA vectors. Viral vectors, and especially retroviral vectors, have become the most widely used method for inserting genes into mammalian, e.g., human cells. Other viral vectors can be derived from lentivirus, poxviruses, herpes simplex virus I, adenoviruses and adeno-associated viruses, and the like. See, for example, U.S. Pat, Nos. 5,350,674 and 5,585,362.

Chemical means for introducing a polynucleotide into a host cell comprise colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle *in vitro* and *in vivo* is a liposome (e.g., an artificial membrane vesicle).

In the case where a non-viral delivery system is utilized, an exemplary delivery vehicle is a liposome. The use of lipid formulations is contemplated for the introduction of the nucleic acids into a host cell (*in vitro, ex vivo* or *in vivo*)*.* In another aspect, the nucleic acid may be associated with a lipid. The nucleic acid associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. Lipid, lipid/DNA or lipid/expression vector associated compositions are not limited to any particular structure in solution. For example, they may be present in a bilayer structure, as micelles, or with a "collapsed" structure. They may also simply be interspersed in a solution, possibly forming aggregates that are not uniform in size or shape. Lipids are fatty substances which may be naturally occurring or synthetic lipids. For example, lipids comprise the fatty droplets that naturally occur in the cytoplasm as well as the class of compounds which contain long-chain aliphatic hydrocarbons and their derivatives, such as fatty acids, alcohols, amines, amino alcohols, and aldehydes.

In one embodiment, the vector is a lentiviral vector.

### Preparation

It is further described a preparation comprising the CAR-T cell, and a pharmaceutically acceptable carrier, diluent or excipient. In one embodiment, the preparation is a liquid preparation. Preferably, the preparation is an injection. Preferably, the concentration of the CAR-T cells in the preparation is 1 × 10³-1×10⁸ cells / ml, more preferably 1 × 10⁴-1×10⁷ cells / ml.

In one embodiment, the preparation may comprises buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. The preparation of the invention is preferably formulated for intravenous administration.

### Therapeutic application

The disclosure comprises therapeutic applications using cells (e.g., T cells) transduced with a lentiviral vector (LV) encoding the expression cassette of the invention. The transduced T cells can target the tumor cell markers CD19 and CD20, synergistically activate T cells, and cause T cell immune responses, thereby significantly increasing the killing efficiency against tumor cells.

Thus, it is also described a method for stimulating a T cell-mediated immune response to a target cell population or tissue in a mammal comprising the step of administering to the mammal a CAR-T cell of the invention.

In one embodiment, it is disclosed a class of cell therapies, wherein T cells from autologous patient (or heterologous donor) are isolated, activated and genetically modified to generate CAR-T cells, and then injected into the same patient. The probability of graft versus host disease in this way is extremely low, and antigens are recognized by T cells in a non-MHC-restricted manner. In addition, one CAR-T can treat all cancers that express the antigen. Unlike antibody therapies, CAR-T cells are able to replicate *in vivo* resulting in long-term persistence that can lead to sustained tumor control.

In one embodiment, the CAR-T cells of the invention can undergo robust in vivo T cell expansion and can persist for an extended amount of time. In addition, the CAR mediated immune response may be part of an adoptive immunotherapy approach in which CAR-modified T cells induce an immune response specific to the antigen binding moiety in the CAR. For example, an anti-CD19CD20 CAR-T cell elicits an immune response specifically against cells expressing CD19 and CD20.

Cancers that may be treated comprise tumors that are unvascularized or largely unvascularized, and tumors that are vascularized. Cancers may comprise non-solid tumors (such as hematological tumors, for example, leukemias and lymphomas) or solid tumors. Types of cancers to be treated with the CARs of the invention comprise, but are not limited to, carcinoma, blastoma, and sarcoma, and certain leukemia or lymphoid malignancies, benign and malignant tumors, and malignancies e.g., sarcomas, carcinomas, and melanomas. Adult tumors/cancers and pediatric tumors/cancers are also comprised.

Hematologic cancers are cancers of the blood or bone marrow. Examples of hematological (or hematogenous) cancers comprise leukemias, including acute leukemias (such as acute lymphocytic leukemia, acute myelocytic leukemia, acute myelogenous leukemia and myeloblasts, promyeiocytic, myelomonocytic, monocytic and erythroleukemia), chronic leukemias (such as chronic myelocytic (granulocytic) leukemia, chronic myelogenous leukemia, and chronic lymphocytic leukemia), polycythemia vera, lymphoma, Hodgkin's disease, non-Hodgkin's lymphoma (indolent and high grade forms), multiple myeloma, Waldenstrom's macroglobulinemia, heavy chain disease, myelodysplastic syndrome, hairy cell leukemia and myelodysplasia.

Solid tumors are abnormal masses of tissue that usually do not contain cysts or liquid areas. Solid tumors can be benign or malignant. Different types of solid tumors are named for the type of cells that form them (such as sarcomas, carcinomas, and lymphomas). Examples of solid tumors, such as sarcomas and carcinomas, comprise fibrosarcoma, myxosarcoma, liposarcoma, mesothelioma, malignant lymphoma, pancreatic cancer and ovarian cancer.

The CAR-modified T cells of the invention may also serve as a type of vaccine for *ex vivo* immunization and/or *in vivo* therapy in a mammal. Preferably, the mammal is a human.

With respect to *ex vivo* immunization, at least one of the following occurs *in vitro* prior to administering the cell into a mammal: i) expaning the cells, ii) introducing a nucleic acid encoding a CAR to the cells, and/or iii) cryopreservation of the cells.

*Ex vivo* procedures are well known in the art and are discussed more fully below. Briefly, cells are isolated from a mammal (preferably a human) and genetically modified (i.e., transduced or transfected *in vitro*) with a vector expressing a CAR disclosed herein. The CAR-modified cell can be administered to a mammalian recipient to provide a therapeutic benefit. The mammalian recipient may be a human and the CAR-modified cell can be autologous with respect to the recipient. Alternatively, the cells can be allogeneic, syngeneic or xenogeneic with respect to the recipient.

In addition to using a cell-based vaccine in terms of *ex vivo* immunization, the present disclosure also provides compositions and methods for *in vivo* immunization to elicit an immune response directed against an antigen in a patient.

It is further described methods for treating tumors comprising administering to a subject in need thereof, a therapeutically effective amount of the CAR-modified T cells of the invention.

The CAR-modified T cells of the present invention may be administered either alone, or as a pharmaceutical composition in combination with diluents and/or with other components such as IL-2, IL-17 or other cytokines or cell populations. Briefly, pharmaceutical compositions of the present invention may comprise a target cell population as described herein, in combination with one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions of the present invention are preferably formulated for intravenous administration.

Pharmaceutical compositions of the present invention may be administered in a manner appropriate to the disease to be treated (or prevented). The quantity and frequency of administration will be determined by such factors as the condition of the patient, and the type and severity of the patient's disease, although appropriate dosages may be determined by clinical trials.

When "an immunologically effective amount", "an anti-tumor effective amount", "an tumor-inhibiting effective amount", or "therapeutic amount" is indicated, the precise amount of the compositions of the present invention to be administered can be determined by a physician with consideration of individual differences in age, weight, tumor size, extent of infection or metastasis, and condition of the patient (subject). It can generally be stated that a pharmaceutical composition comprising the T cells described herein may be administered at a dosage of 10⁴ to 10⁹ cells/kg body weight, preferably 10⁵ to 10⁶ cells/kg body weight, including all integer values within those ranges. T cell compositions may also be administered multiple times at these dosages. The cells can be administered by using infusion techniques that are commonly known in immunotherapy (see, e.g., Rosenberg et al, New Eng. J. of Med. 319: 1676, 1988). The optimal dosage and treatment regime for a particular patient can readily be determined by one skilled in the art of medicine by monitoring the patient for signs of disease and adjusting the treatment accordingly.

The administration of the subject compositions may be carried out in any convenient manner, including by aerosol inhalation, injection, ingestion, transfusion, implantation or transplantation. The compositions described herein may be administered to a patient subcutaneously, intradermaliy, intratumorally, intranodally, intramedullary, intramuscularly, by intravenous (*i.v*.) injection, or intraperitoneally. In one embodiment, the T cell compositions of the present invention are administered to a patient by intradermal or subcutaneous injection. In another embodiment, the T cell compositions of the present invention are preferably administered by i.v. injection. The compositions of T cells may be injected directly into a tumor, lymph node, or site of infection.

In certain embodiments of the present invention, cells activated and expanded using the methods described herein, or other methods known in the art where T cells are expanded to therapeutic levels, are administered to a patient in conjunction with (e.g., before, simultaneously or following) any number of relevant treatment modalities, including but not limited to treatment with agents such as antiviral therapy, cidofovir and interleukin-2, Cytarabine (also known as ARA-C) or natalizumab treatment for MS patients or efalizumab treatment for psoriasis patients or other treatments for PML patients. In further embodiments, the T cells of the invention may be used in combination with chemotherapy, radiation, immunosuppressive agents, such as cyclosporin, azathioprine, methotrexate, mycophenolate, and FK506, antibodies, or other immunotherapeutic agents. In a further embodiment, the cell compositions of the present invention are administered to a patient in conjunction with (e.g., before, simultaneously or following) bone marrow transplantation, or the use of chemotherapy agents such as, fludarabine, external-beam radiation therapy (XRT), cyclophosphamide. For example, in one embodiment, subjects may undergo standard treatment with high dose chemotherapy followed by peripheral blood stem cell transplantation. In certain embodiments, following the transplant, subjects receive an infusion of the expanded immune cells of the present invention. In an additional embodiment, expanded cells are administered before or following surgery.

The dosage of the above treatments to be administered to a patient will vary with the precise nature of the condition being treated and the recipient of the treatment. The scaling of dosages for patient administration can be performed according to art-accepted practices. In general, 1×10⁶ to 1×10¹⁰ of the modified T cells of the invention (e.g., CAR-T-19/20 cells) can be applied to patients by means of, for example, intravenous infusion each treatment or each course of treatment.

The advantages of the present invention are:
(1) As for the chimeric antigen receptor of the present invention, the extracellular antigen binding domain is specific anti-CD20 scFv and anti-CD19 scFv; the CAR formed by combining the specific anti-CD20 scFv and anti-CD19 scFv to a specific hinge region and an intracellular domain shows a great ability of killing tumor cells with low cytotoxicity and low side effects.
(2) The chimeric antigen receptor provided by the invention can achieve stable expression and membrane localization of CAR protein after T cells are infected by lentivirus carrying CAR gene.
(3) The CAR-modified T cell of the present invention has a longer survival time *in vivo* and strong anti-tumor efficacy; the optimized CAR with the IgG4 Hinge-CH2-CH3 linker region can avoid the binding of the Fc receptor and the subsequent ADCC effect (antibody-dependent cytotoxicity).
(4) Compared with two independent CARs, the bispecific chimeric antigen receptor of the present invention comprises both anti-CD20 scFv and anti-CD19 scFv, and the DNA footprint is significantly reduced (the DNA length is reduced by 40%), and the size of structure is small, which is beneficial for the packaging and transduction efficiency of viral vectors, thus directly improving clinical efficacy. In addition, the bispecific CAR of the invention has lower cost, higher cure rate, and more safety.
(5) The T-cells modified with TN-LEU-19 or TH-OF-19 chimeric antigen receptors have very strong ability to kill *in vitro* and to clear malignant tumors carrying CD19 and CD20 positive antigens *in vivo,* and Ofatumumab is stronger. This provides a new effective method and preparation for the clinical application of CAR-T in the treatment of CD19 and CD20-positive leukemias and lymphomas.
(6) The CAR-T cells of the present invention have a killing effect on most malignant B-cell tumors, have a wider treatment range and a larger coverage rate, and can more effectively prevent tumor cells from escaping.

The present invention will be further illustrated below with reference to the specific examples. It is to be understood that these examples are for illustrative purposes only and are not intended to limit the scope of the invention. The experimental methods with no specific conditions described in the following examples are generally performed under the conventional conditions, or according to the manufacturer's instructions. Percentages and parts are by weight unless otherwise stated.

### Example 1 Construction of lentiviral expression vector

The full-length DNA was synthesized and cloned to construct the encoding plasmids. The pWPT lentiviral vector was selected as a cloning vector, and the cloning sites were BamH I and Sal I sites. Wherein, the structures of the two CARs designed in the present invention are shown in Figure 1. The amino acid sequence of TN-LEU-19 is shown in SEQ ID NO: 15, and the amino acid sequence of TN-OF-19 is shown in SEQ ID NO: 16 (with a structure of L-(OF)VL-(OF)VH-I-(FMC63)VH-(FMC63)VL-H-TM-C-CD3ζ).

### Example 2 Detection of the in vitro activation ability of bi-specific CARs

PBMCs were isolated from venous blood of healthy donor by density gradient centrifugation. On day 0, PBMCs were activated in a cell culture flask previously coated with CD3 monoclonal antibody (OKT3) and Retronectin (TAKARA). The medium was GT-551 cell culture medium containing 1% human albumin and 300 U/mL recombinant human interleukin 2 (IL-2). On day 3, activated PBMCs were transduced with purified TN-LEU-19 or TN-OF-19 lentivirus solution. Starting from day 6, TN-OF-19 and TN-LEU-19 CAR-T cells can be taken for corresponding activity assay. Protein L method was used to determine the expression level of CAR gene-encoded protein on the surface of T cell membrane in CAR-T cells cultured for 7 days.

T cell activation marker CD137 and IFNγ release were detected using CD19/20 bi-specific CAR-T cells cultured for 7 days. 1×10⁵ of CAR-T cells were co-cultured respectively with CD19, CD20-positive K562-CD19+, K562-CD20+, K562-CD19+CD20+ and Raji (naturally expressing CD19 and CD20) tumor cell line, as well as CD19CD20-negative K562 tumor cell line, or without tumor cells, in 200 µl of GT-551 medium for 18h with a ratio of 1:1. Then the expression level of CD137 on the surface of T cell and the secretion level of IFNγ in the culture supernatant were detected respectively.

The results are shown in Figures 2A and 2B. The expressions of CD137 on the surface of two CART cells were detected, and the expressions of IFNγ in the culture supernatants were detected. Wherein, TN-OF-19 cells has higher CD137 activation level and IFNγ release level than TN-LEU-19.

### Example 3 Identification of the optimal antigen binding domain for anti-CD19/CD20 bispecific CAR

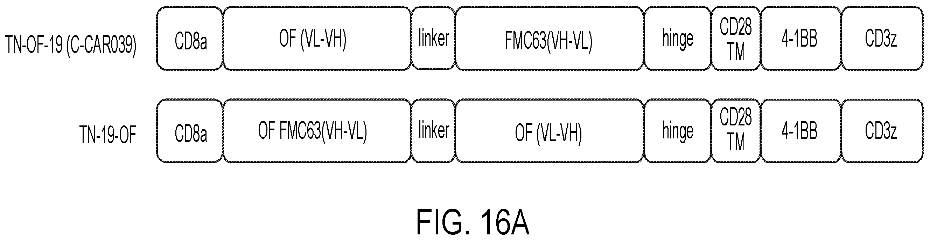

We prepared two bispecific CARs having the same anti-CD20 scFv and anti-CD19 scFv but in different orders: TN-OF-19 (C-CAR039) has anti-CD20 scFv ("OF") followed by anti-CD19 scFv ("FMC63"), while TN-19-OF has anti-CD19 scFv ("FMC63") followed by anti-CD20 scFv ("OF") (Figure 16A).

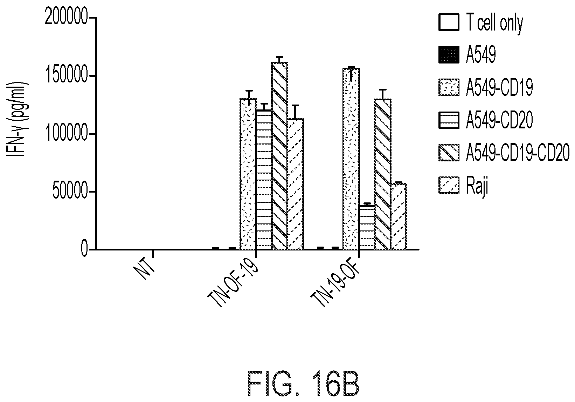

As shown in Figure 16B, TN-OF-19 (C-CAR039) demonstrated higher *in vitro* activities (e.g., inducing interferon-γ or IFN-γ release) against the CD19 and CD20-positive cells than CAR-T20-29 (V_{L}-V_{H}).

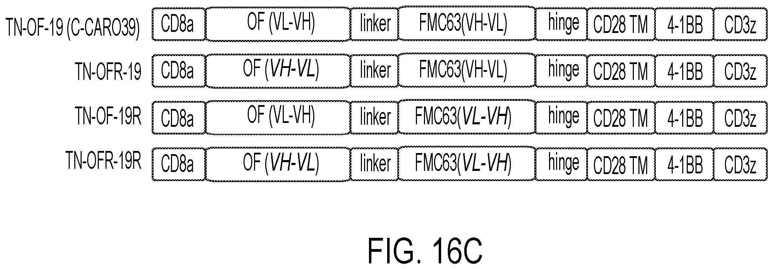

Additionally, we prepared different bispecific CARs having the same anti-CD20 scFv and anti-CD19 scFv order (i.e., anti-CD20 scFv ("OF") followed by anti-CD19 scFv ("FMC63")), but with different V_{H} and V_{L} order in either the anti-CD20 scFv (OF) or the anti-CD19 scFv (FMC63) (Figure 16C).

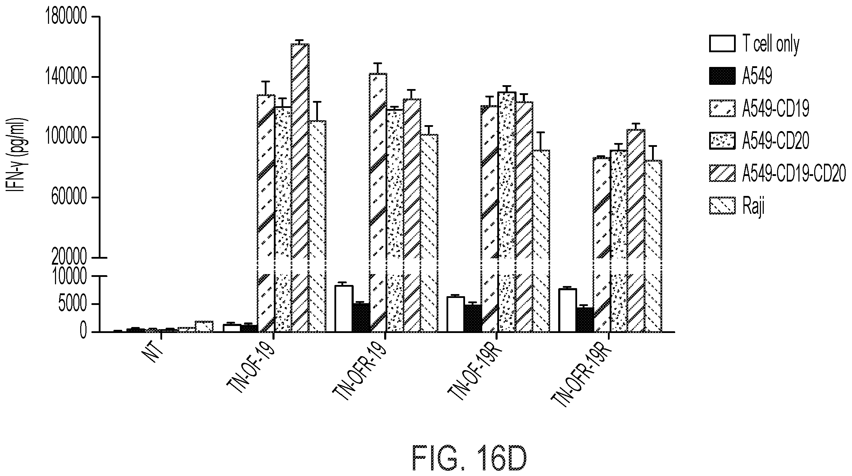

As shown in Figure 16D, TN-OF-19 (C-CAR039) demonstrated superior anti-tumor activities compared with the other bispecific CARs with different V_{H} and V_{L} orders.

Our *in vitro* data show that the order of the anti-CD20 scFv and anti-CD19 scFv in the bispecific CAR, as well as the order of V_{H} and V_{L} in a scFv, can affect the properties of the bispecific CAR.

### Example 4 Detection of cytotoxicity of CD19/20 bi-specific CAR-T cells in vitro

The CAR-T cells prepared in Example 2 were tested for cytotoxicity by LDH release assay. Target cells are K562, K562-CD19+, K562-CD20+, K562-CD19+CD20+, and Raji cells; and effector cells are NT, TN-LEU-19, and TN-OF-19 cells. The effect-target ratio was set, wherein number of effector cells: number of target cells = 5: 1, 10: 1, 20: 1, and 40: 1. The results are shown in Figure 3A. Both TN-LEU-19 and TN- OF-19 CAR-T cells can well induce apoptosis and release LDH in CD19/20-positive tumor cells.

CD107a (a marker of T-Cell Degranulation) release levels during tumor cell killing induced by CAR-T19/20s cells were analyzed by flow cytometry. 1×10⁵ cells of effective cell CAR-T19/20s (TN-OF-19 and TN-LEU-19) were co-cultured respectively with 2×10⁵ of target tumor cells. The target cells are K562-CD19+, K562-CD20+, K562-CD19+CD20+, and K562 cells, Raji cells, Romas cells (CD19+CD20+), respectively. The results are shown in Figure 3B. Both CART cells can well induce the release of CD107a during tumor cell killing. Wherein, TN-OF-19 cells have a slightly higher release of CD107a as a marker of cytotoxicity than TN-LEU-19 cells.

### Example 5 Inhibitory effect of CAR-T19/20s on transplanted tumor cells in mice

The tumor cells injected in animals were Raji cells carrying a luciferase reporter gene (Raji expressing luciferase). In this experiment, tumor cells Raji were injected and grown in mice for one week, and then effector T cells were injected. The effector T cells were divided into three groups: NT, TN-LEU-19, and TN-OF-19. The expanded effector T cells were injected into the NSG mice through the tail vein, and then the fluorescence intensity of the mice (via IVIS fluorescence imaging) and the weight of the mice were recorded every 7 days. The experiment was stopped on the 21st day, and the statistical results were analyzed.

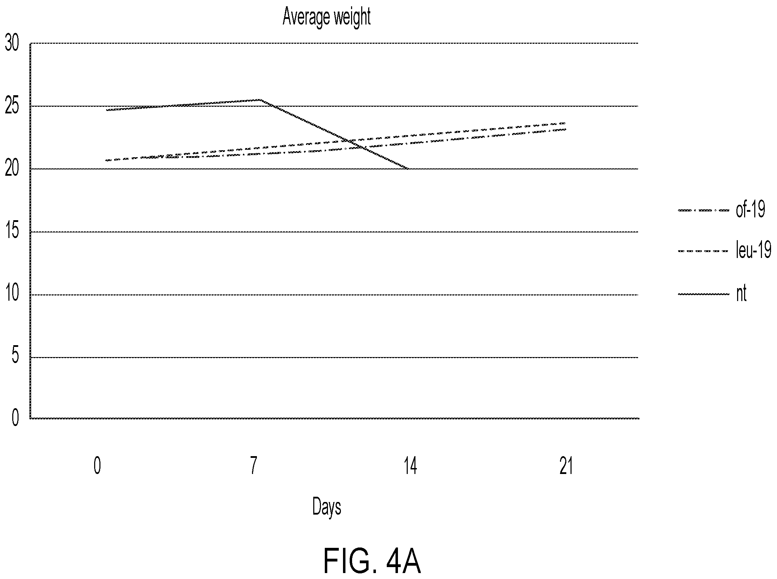

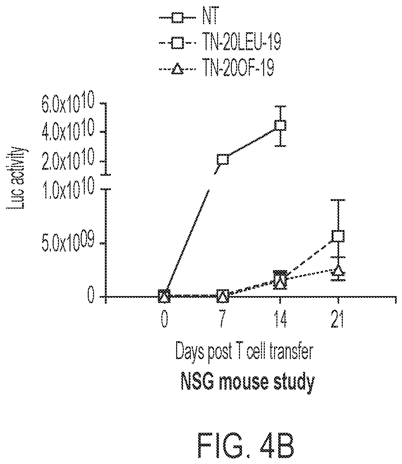

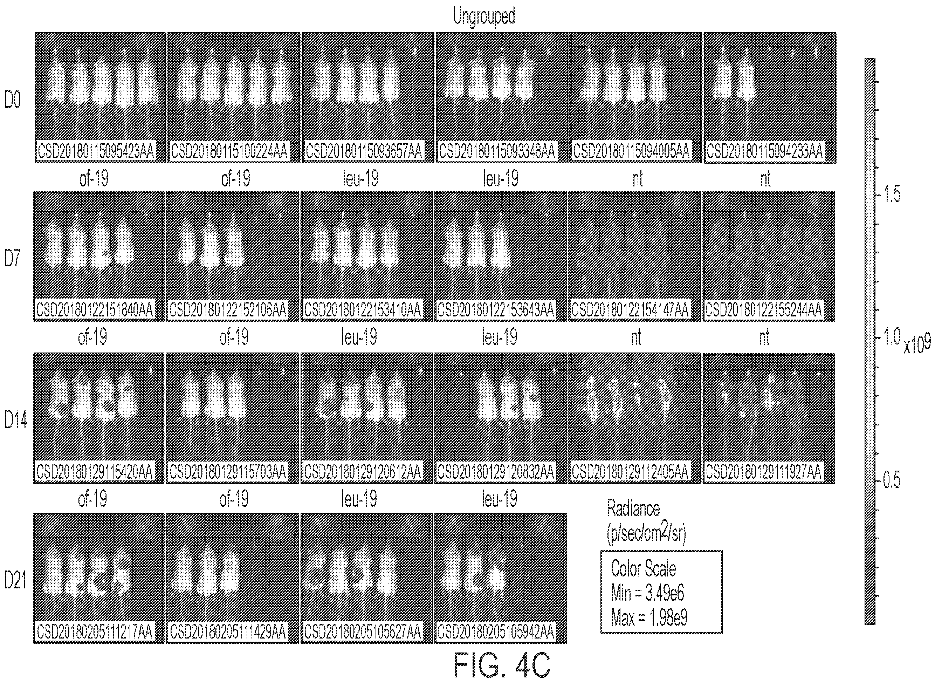

The results are shown in Figures 4A-4C. Figure 4A shows the weight change of mice after the injection of effector T cells into the three groups of mice. Compared with two types of CART cells CART-TN-OF-19 and CART-TN-LEU-19, the mice of NT showed a significant decrease in body weight and the mice in both CART cell groups gained slightly more weight. For *in vivo* studies, NSG mice were xenografted with Raji-Luc cells which are human Burkitt's lymphoma Raji cells expressing firefly luciferase as a reporter. Different CAR-T cells or a negative control were then administered to the mice. The fluorescence intensity of the animals xenografted with Raji-Luc were assayed after treatment, which reflected the proliferation of tumor cells in the animals. Figure 4B shows the average fluorescence intensity of the three groups of mice. The results show that the average fluorescence intensity of the mice in the NT group increased significantly, while the average of the fluorescence intensities of the mice in the two CART cell groups decreased, indicting both TN-OF-19 and TN-LEU-19 CART cells can inhibit the growth of tumor compared with NT. Wherein, TN-OF-19 cells have better inhibition on tumor growth than TN-LEU-19 cells after day14, and the tumor growth curve of TN-LEU-19 group relapsed significantly. Figure 4C shows the IVIS imaging of fluorescence intensity in two CART cell groups and NT group. TN-OF-19 CAR-T cells can inhibit or kill tumor cells better than TN-LEU-19 cells *in vivo.*

Thus, comparing the CD20/CD19 bi-CAR with Leu16 scFv (used in the clinical studies by Nirav NS or Tong C), C-CAR039 showed superior anti-tumor activity against both single positive and double positive tumor targets *in vitro* and *in vivo.*

### Example 6 Comparison of inhibitory effect of TN-OF-19 CAR-T cells and CD19 specific CAR-T cells in vivo

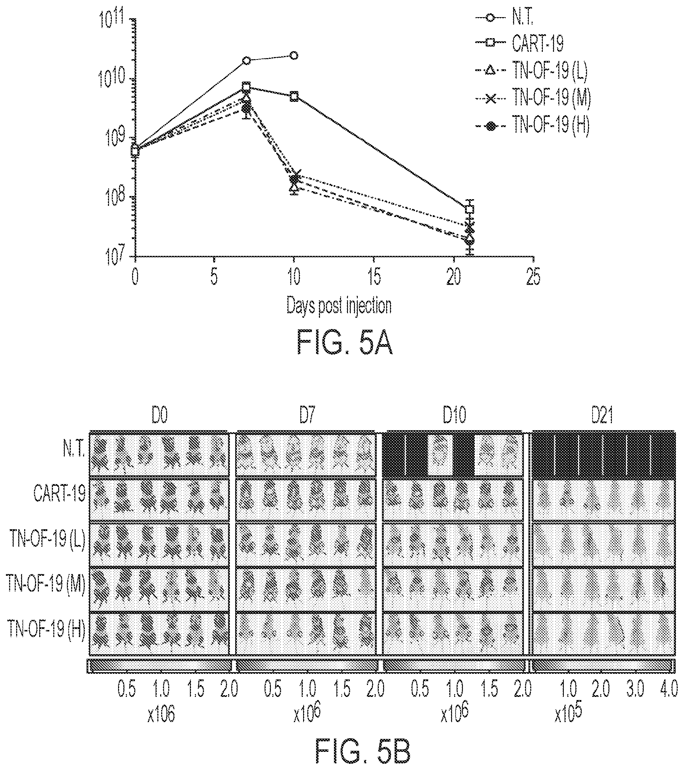

1×10⁶ firefly luciferase expressing Raji cells were administered to 6- to 8-week-old NPG mice (NOD-Cg.PrkdcSCID IL-2Rgcnull/vst) via tail vein injection. Seven days later, tumor engraftment was measured by i.p. injection of D-luciferin and imaging 10-15 minutes later for 180 seconds on a Bruker In Vivo Xtreme imaging system (Bruker, Xtreme BI). Mice were distributed equally to study groups (n=6/group) based on tumor burden. The TN-OF-19(L), TN-OF-19(M), TN-OF-19(H) groups were treated separately with 1×10⁶, 2.5×10⁶, and 5×10⁶ CAR+ T cells via tail vein injection. 1×10⁶ non-transduced T cells (N.T.) and single target CD19 specific CAR-T cells from the same donor served as controls. Tumor growth was assessed based on mouse whole body average radiance on days 7, 10, 21 following injection. Figure 5A shows that bi-specific TN-OF-19 CAR-T cells can inhibit or kill tumor cells better than CD19 specific CAR-T cells *in vivo.* Especially at early stage of treatment, bi-specific TN-OF-19 CAR-T shows significantly faster inhibitory effects. Figure 5B shows the IVIS imaging of fluorescence intensity in 4 CAR-T cell groups and NT group.

### Example 7 Antigen specificity of the CD20/CD19 bi-specific CAR

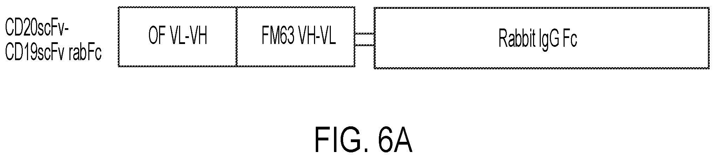

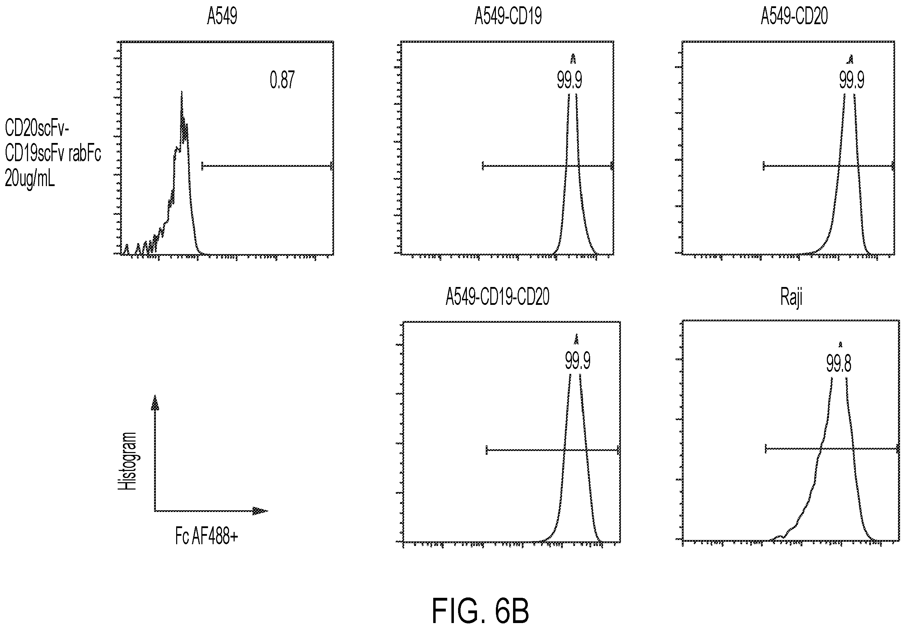

To examine the affinity and specificity of the bi-specific scFv in TN-OF 19 CAR, a chimeric rabbit monoclonal antibody was generated by linking the CD20 scFv (e.g., derived from the Ofatumumab mAb) and the CD19 scFv (e.g., derived from the FMC63 mAb) in frame with a rabbit IgG1 Fc region. CD19 and CD20 scFvs were alternatively linked by a G4S linker. The molecular order is OF VL-VH-G4S-FMC63 VH-VL, which is the same order as in the scFVs in TN-OF-19. The chimeric antibody was expressed in 293T cells after transient transfection. Validation of this chimeric antibody for specificity of staining was performed by flow cytometry. Briefly, three stable cell lines (A549-CD19, A549-CD20, A549-CD19-CD20) were used as targets, and CD19-CD20-A549 cells and CD19+CD20+ Raji cells were used as control. All cells were washed and resuspended, blocking by 2% serum for 30 minutes. 5×10⁵ cells were transferred to FACS vials, washed and stained with recombinant antibodies (final concentration 20 µg/mL) for 1 h at 4°C. After washing, the secondary antibody (goat anti-rabbit IgG) was added for 30 minutes in dark at 4°C. Finally, cells were washed and resuspended in 200 µL FACS buffer for FACS analysis. Figure 6B shows the chimeric antibody bound to cells with CD19 or CD20 or both, but not to cells lacking both antigens, indicating that the bispecific binding domain required only one cognate antigen for binding and no new specific recognition sites were formed.

Tissue cross reactivity and whole genome membrane proteome array studies confirmed the binding specificity of C-CAR039.

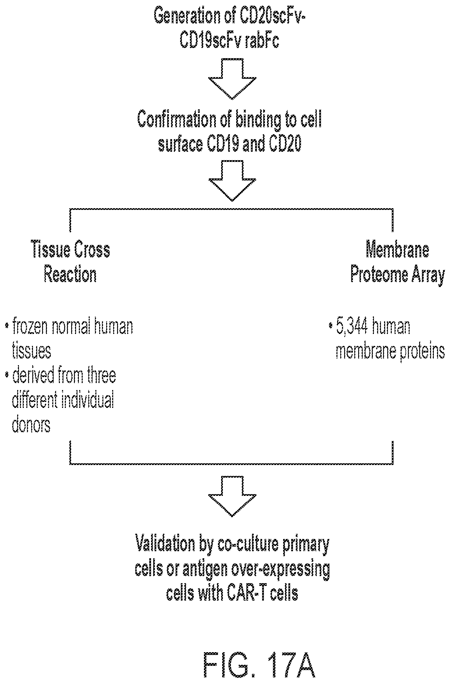

Figure 17A shows the process of evaluating binding specificity of the antigen binding domain of C-CAR039. Table 1 shows that strong specific membrane staining was observed on human lymphocytes in blood, bone marrow, lymph node, spleen, thymus (IHC GLP study).

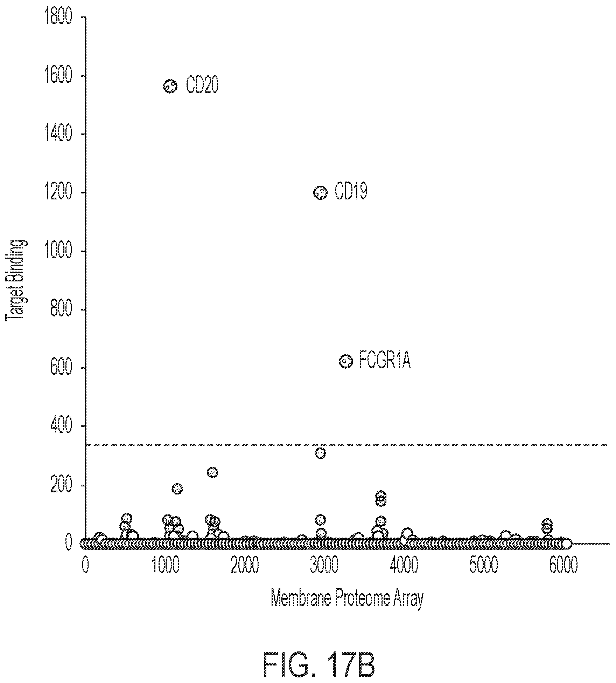

CD19, CD20 and FCGR1A were identified with moderate binding at the concentrations of 5.0 µg/mL, no other significant interactions were identified. FCGR1 was reported to bind rabFc (Figure 17B).

**Table 1**

| Tissue | 2.0 µg/mL | | | 10.0 µg/mL | | |
|---|---|---|---|---|---|---|
| | Donor 1 | Donor 2 | Donor 3 | Donor 1 | Donor 2 | Donor 3 |
| Cytomembrane of scattered lymphocytes in bladder | 2-1^{a} | 3-3 | 2-1 | 2-1 | 3-3 | 2-1 |
| Cytomembrane of lymphocytes in blood cells | 2-1 | 2-1 | 2-1 | 2-1 | 2-1 | 2-1 |
| Cytomembrane of lymphocytes in bone marrow | 1-2 | 1-2 | 1-2 | 2-3 | 2-3 | 2-3 |
| Cytomembrane of lymphocytes in lymph node | 2-2 | 2-2 | 3-2 | 3-3 | 3-3 | 3-3 |
| Cytomembrane of splenic lymphocytes | 2-2 | 2-2 | 2-2 | 3-2 | 3-2 | 3-2 |
| Cytomembrane of thymic lymphocytes | 1-1 | 2-3 | 0-0 | 2-2 | 3-3 | 1-1 |

| | | | | | | |
|---|---|---|---|---|---|---|
| a. positive intensity - percentage of specific staining. 0, no stained cells; 1, Minimal or equivocal staining; 2, Weakly positive staining; 3, moderately positive staining. 0, no stained cells; 1, <25% cells are stained; 2, 25-50% cells are stained; 3, 50%-75% cells are stained. | | | | | | |

### Example 8 Screening and functional verification of CD20 specific CARs (for making CD20/19 bi-specific CARs)

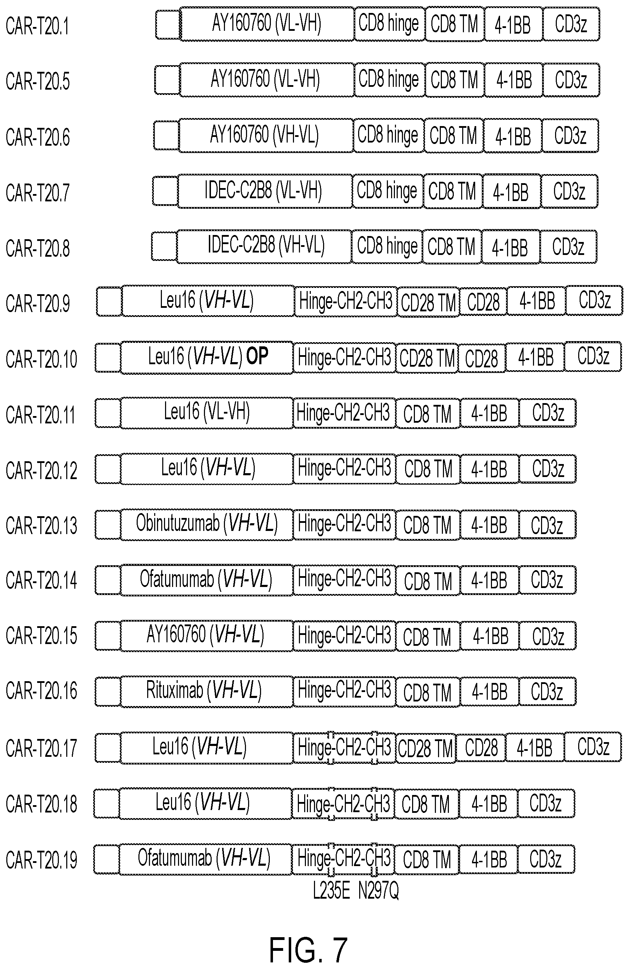

Before the construction of the CD20/CD19 bi-specific CARs, we did extensive studies to screen and narrow down the CD 20 specific scFV candidates. Figure 7 shows the structures of sixteen CD20 specific CARs with six different scFVs and different hinge/TM/signaling domains. Full-length DNA was synthesized and cloned to achieve the construction of encoding plasmids, and we tested the anti-tumor activities of these CARs with various CD20 expressing target cells.

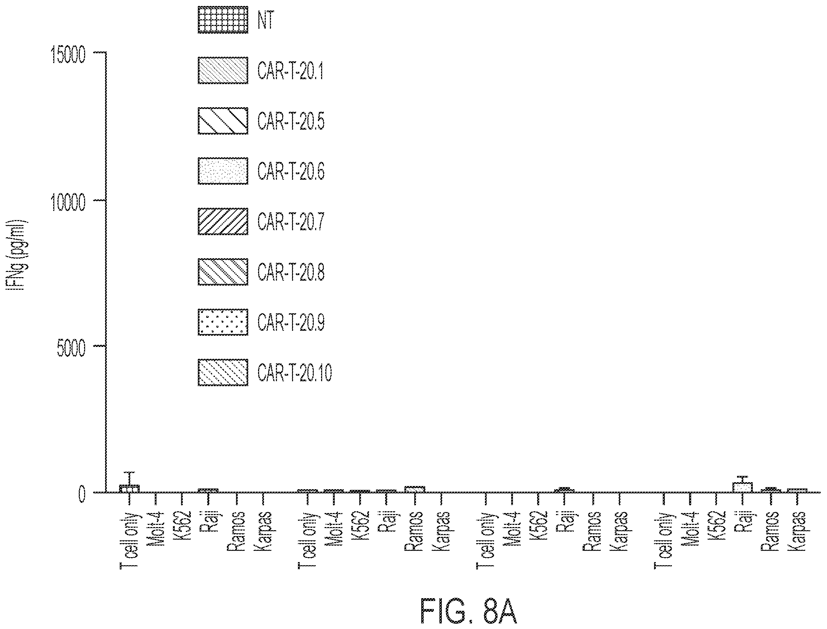

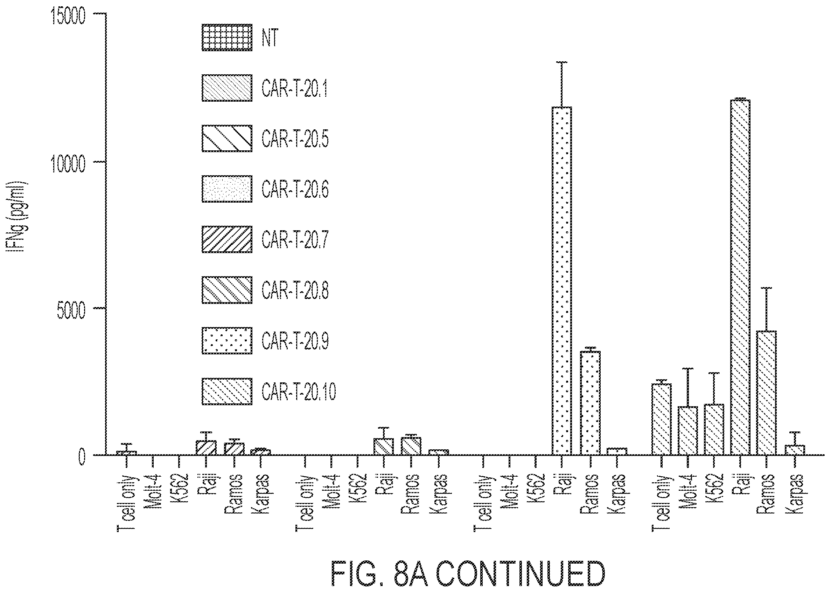

Figure 8A shows the results of IFNγ release assay to screen CAR-T20.1, CAR-T20.5, CAR-T20.6, CAR-T20.7, CAR-T20.8, CAR-T20.9 and CAR-T20.10, and among these CAR-Ts, only CAR-T20.9 (Leu16) and CAR-T20.10 (Leu16) showed higher positive IFN□ release.

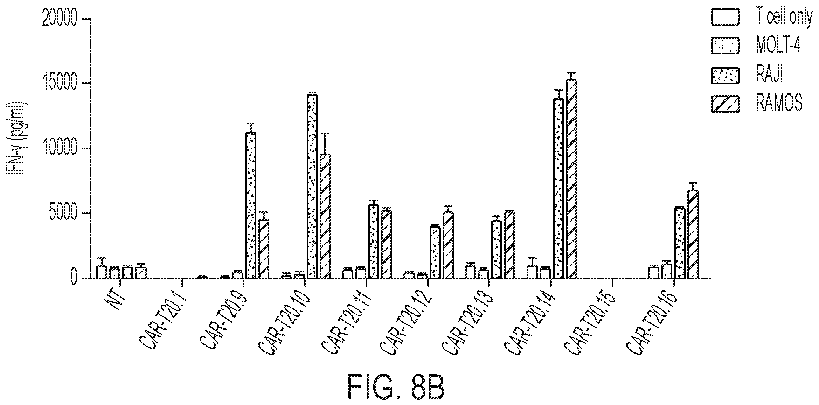

Figure 8B shows the results of IFNγ release assay to screen CAR-T20.1, CAR-T20.9, CAR-T20.10, CAR-T20.11, CAR-T20.12, CAR-T20.13, CAR-T20.14, CAR-T20.15 and CAR-T20.16. Among these CAR-Ts, CAR-T20.10 (Leu16) and CAR-T20.14 (OF) showed higher positive IFNγ release.

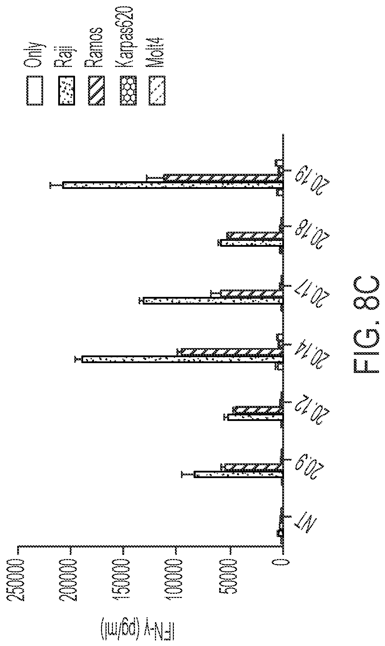

Figure 8C shows the results of IFNγ release assay to screen CAR-T20.9, CAR-T20.12, CAR-T20.14, CAR-T20.17, CAR-T20.18, and CAR-T20.19. Among these CAR-Ts, CAR-T20.14 (OF) and CAR-T20.19 (OF) showed higher positive IFNγ release.

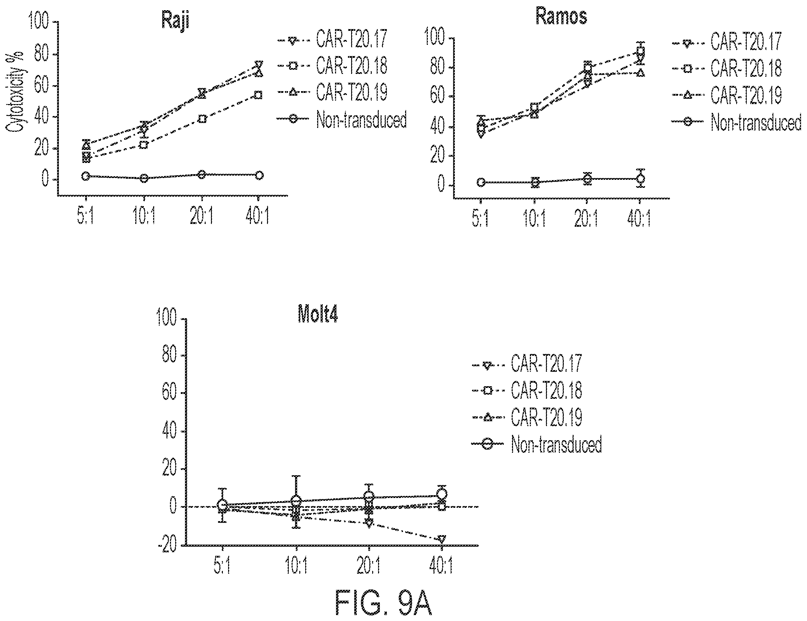

Figure 9A shows the results of CAR-T20.17 (Leu16 3^{rd} generation), CAR-T20.18 (Leu16 2^{nd} generation), CAR-T20.19 (OF 2^{nd} generation) cells tested for cytotoxicity by LDH release assay. Target cells are CD20 positive cell lines Raji and Ramos, and CD20 negative Molt4. Both All three CD20 CAR-T cells can induce apoptosis and release LDH in CD20-positive tumor cells indicating CAR-T20.17, CAR-T20.18, and CAR-T20.19 had strong killing effects on target cells CD20-positive Raji and Romas cells.

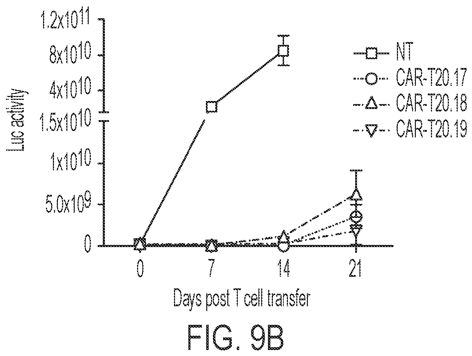

Figure 9B shows the *in vivo* inhibition of tumor growth in NSG mouse studies. The tumor cells injected in animals are Raji expressing luciferase. In this experiment, tumor cells Raji were injected and grown in mice for one week, and then effector T cells were injected through the tail vein, and then the fluorescence intensity of the mice (via IVIS fluorescence imaging) and the weight of the mice were recorded every 7 days. The experiment was stopped on the 21st day, and the statistical results were analyzed. The results show that CAR-T20.19 (OF) cells have better inhibition on tumor growth than CAR-T20.17 (Leu16) and CAR-T201.8 (Leu16) cells after day14.

In summary, through a large number of experiments and comparison, it was found that 20.1, 20.2, 20.4, 20.5, 20.6, 20.7, 20.8 and 20.15 were basically invalid, and 20.11, 20.12 and 20.13 had certain effects, but the effects of them were less than that of 20.9, 20.10, 20.14, 20.16, 20.17, 20.18 and 20.19, wherein the effects of 20.19(OF) were the best. Based on the above structures, CD20 scFv (OF) and CD19 scFv (FMC63) were tandemly used in a new bispecific chimeric antigen receptor TN-OF-19 and considered as the best candidate for further analysis.

The amino acid sequences of the CD20 specific CARs involved in Example 7 are shown in Table 2.

**Table 2 Chimeric antigen receptors and sequences thereof**

| CAR-T | Sequence | SEQ ID NO: |
|---|---|---|
| CAR-T 20.1 | MDIQLTQSPAILSASPGEKVTMTCRASSSLSFMHWYQQKPGSSPKPWIY ATSNLASGVPARFSGSGSGTSYSLTISTVEAEDAASYFCHQWSSNPLTFG AGTKLEISSGGGGSGGGGSGDVMGVDSGGGLVQPGGSRKLSCAAPGFT FSSFGMHWVRQAPEKGLEWVAYISSPSSTLHYADRVKGRFTISRDNPKN TLFLQMKLPSLCYGLLGPRDHVHRLLKTRLSNSIMYFSHFVPVFLPAKP TTTPAPRPPTPAPTIASQPLSLRPEACRPAAGGAVHTRGLDFACDIYIWA PLAGTCGVLLLSLVITLYCRSKRGRKKLLYIFKQPFMRPVQTTQEEDGC SCRFPEEEEGGCELEFRVKFSRSADAPAYQQGQNQLYNELNLGRREEY DVLDKRRGRDPEMGGKPRRKNPQEGLYNELQKDKMAEAYSEIGMKGE RRRGKGHDGLYQGLSTATKDTYDALHMQALPPR | 23 |
| CAR-T 20.5 | MALPVTALLLPLALLLHAARPDIQLTQSPAILSASPGEKVTMTCRASSSL SFMHWYQQKPGSSPKPWIYATSNLASGVPARFSGSGSGTSYSLTISTVE AEDAASYFCHQWSSNPLTFGAGTKLEIGGGGSGGGGSGGGGSDVMGV DSGGGLVQPGGSRKLSCAAPGFTFSSFGMHWVRQAPEKGLEWVAYISS PSSTLHYADRVKGRFTISRDNPKNTLFLQMKLPSLCYGLLGPRDHVHRL LTRTTTPAPRPPTPAPTIASQPLSLRPEACRPAAGGAVHTRGLDFACDIYI WAPLAGTCGVLLLSLVITLYCRSKRGRKKLLYIFKQPFMRPVQTTQEED GCSCRFPEEEEGGCELEFELGTFKTNDLQGSCRS | 24 |
| CAR-T 20.6 | MALPVTALLLPLALLLHAARPDVMGVDSGGGLVQPGGSRKLSCAAPGF TFSSFGMHWVRQAPEKGLEWVAYISSPSSTLHYADRVKGRFTISRDNPK NTLFLQMKLPSLCYGLLGPRDHVHRLLGGGGSGGGGSGGGGSDIQLTQ SPAILSASPGEKVTMTCRASSSLSFMHWYQQKPGSSPKPWIYATSNLAS GVPARFSGSGSGTSYSLTISTVEAEDAASYFCHQWSSNPLTFGAGTKLEI TRTTTPAPRPPTPAPTIASQPLSLRPEACRPAAGGAVHTRGLDFACDIYI WAPLAGTCGVLLLSLVITLYCRSKRGRKKLLYIFKQPFMRPVQTTQEED GCSCRFPEEEEGGCELEFELGTFKTNDLQGSCRS | 25 |
| CAR-T 20.7 | MALPVTALLLPLALLLHAARPQIVLSQSPAILSASPGEKVTMTCRASSSV SYIHWFQQKPGSSPKPWIYATSNLASGVPVRFSGSGSGTSYSLTISRVEA EDAATYYCQQWTSNPPTFGGGTKLEIKGGGGSGGGGSGGGGSQVQLQ QPGAELVKPGASVKMSCKASGYTFTSYNMHWVKQTPGRGLEWIGAIY PGNGDTSYNQKFKGKATLTADKSSSTAYMQLSSLTSEDSAVYYCARST YYGGDWYFNVWGAGTTVTVSATRTTTPAPRPPTPAPTIASQPLSLRPEA CRPAAGGAVHTRGLDFACDIYIWAPLAGTCGVLLLSLVITLYCRSKRGR KKLLYIFKQPFMRPVQTTQEEDGCSCRFPEEEEGGCELEFELGTFKTNDL QGSCRS | 26 |
| CAR-T 20.8 | MALPVTALLLPLALLLHAARPQVQLQQPGAELVKPGASVKMSCKASG YTFTSYNMHWVKQTPGRGLEWIGAIYPGNGDTSYNQKFKGKATLTAD KSSSTAYMQLSSLTSEDSAVYYCARSTYYGGDWYFNVWGAGTTVTVS AGGGGSGGGGSGGGGSQIVLSQSPAILSASPGEKVTMTCRASSSVSYIH WFQQKPGSSPKPWIYATSNLASGVPVRFSGSGSGTSYSLTISRVEAEDA ATYYCQQWTSNPPTFGGGTKLEIKTRTTTPAPRPPTPAPTIASQPLSLRPE ACRPAAGGAVHTRGLDFACDIYIWAPLAGTCGVLLLSLVITLYCRSKRG RKKLLYIFKQPFMRPVQTTQEEDGCSCRFPEEEEGGCELEFELGTFKTND LQGSCRS | 27 |
| CAR-T 20.9 | MALPVTALLLPLALLLHAARPEVQLQQSGAELVKPGASVKMSCKASGY TFTSYNMHWVKQTPGQGLEWIGAIYPGNGDTSYNQKFKGKATLTADK SSSTAYMQLSSLTSEDSADYYCARSNYYGSSYWFFDVWGAGTTVTVSS GGGGSGGGGSGGGGSDIVLTQSPAILSASPGEKVTMTCRASSSVNYMD WYQKKPGSSPKPWIYATSNLASGVPARFSGSGSGTSYSLTISRVEAEDA ATYYCQQWSFNPPTFGGGTKLEIKESKYGPPCPPCPAPEFLGGPSVFLFP PKPKDTLMISRTPEVTCVVVDVSQEDPEVQFNWYVDGVEVHNAKTKPR EEQFNSTYRVVSVLTVLHQDWLNGKEYKCKVSNKGLPSSIEKTISKAKG QPREPQVYTLPPSQEEMTKNQVSLTCLVKGFYPSDIAVEWESNGQPENN YKTTPPVLDSDGSFFLYSRLTVDKSRWQEGNVFSCSVMHEALHNHYTQ KSLSLSLGKFWVLVVVGGVLACYSLLVTVAFIIFWVRSKRSRLLHSDY MNMTPRRPGPTRKHYQPYAPPRDFAAYRSKRGRKKLLYIFKQPFMRPV QTTQEEDGCSCRFPEEEEGGCELRVKFSRSADAPAYKQGQNQLYNELN LGRREEYDVLDKRRGRDPEMGGKPRRKNPQEGLYNELQKDKMAEAYS EIGMKGERRRGKGHDGLYQGLSTATKDTYDALHMQALPPR | 28 |
| CAR-T 20.10 | MALPVTALLLPLALLLHAARPEVQLQQSGAELVKPGASVKMSCKASGY TFTSYNMHWVKQTPGQGLEWIGAIYPGNGDTSYNQKFKGKATLTADK SSSTAYMQLSSLTSEDSADYYCARSNYYGSSYWFFDVWGAGTTVTVSS GGGGSGGGGSGGGGSDIVLTQSPAILSASPGEKVTMTCRASSSVNYMD WYQKKPGSSPKPWIYATSNLASGVPARFSGSGSGTSYSLTISRVEAEDA ATYYCQQWSFNPPTFGGGTKLEIKESKYGPPCPPCPAPEFLGGPSVFLFP PKPKDTLMISRTPEVTCVVVDVSQEDPEVQFNWYVDGVEVHNAKTKPR EEQFNSTYRVVSVLTVLHQDWLNGKEYKCKVSNKGLPSSIEKTISKAKG QPREPQVYTLPPSQEEMTKNQVSLTCLVKGFYPSDIAVEWESNGQPENN YKTTPPVLDSDGSFFLYSRLTVDKSRWQEGNVFSCSVMHEALHNHYTQ KSLSLSLGKFWVLVVVGGVLACYSLLVTVAFIIFWVRSKRSRLLHSDY MNMTPRRPGPTRKHYQPYAPPRDFAAYRSKRGRKKLLYIFKQPFMRPV QTTQEEDGCSCRFPEEEEGGCELRVKFSRSADAPAYKQGQNQLYNELN LGRREEYDVLDKRRGRDPEMGGKPRRKNPQEGLYNELQKDKMAEAYS EIGMKGERRRGKGHDGLYQGLSTATKDTYDALHMQALPPR | 29 |
| CAR-T 20.11 | MALPVTALLLPLALLLHAARPDIVLTQSPAILSASPGEKVTMTCRASSSV NYMDWYQKKPGSSPKPWIYATSNLASGVPARFSGSGSGTSYSLTISRVE AEDAATYYCQQWSFNPPTFGGGTKLEIKGGGGSGGGGSGGGGSEVQL QQSGAELVKPGASVKMSCKASGYTFTSYNMHWVKQTPGQGLEWIGAI YPGNGDTSYNQKFKGKATLTADKSSSTAYMQLSSLTSEDSADYYCARS | 30 |
| | NYYGSSYWFFDVWGAGTTVTVSSESKYGPPCPPCPAPEFLGGPSVFLFP PKPKDTLMISRTPEVTCVVVDVSQEDPEVQFNWYVDGVEVHNAKTKPR EEQFNSTYRVVSVLTVLHQDWLNGKEYKCKVSNKGLPSSIEKTISKAKG QPREPQVYTLPPSQEEMTKNQVSLTCLVKGFYPSDIAVEWESNGQPENN YKTTPPVLDSDGSFFLYSRLTVDKSRWQEGNVFSCSVMHEALHNHYTQ KSLSLSLGKIYIWAPLAGTCGVLLLSLVITLYCKRGRKKLLYIFKQPFMR PVQTTQEEDGCSCRFPEEEEGGCELRVKFSRSADAPAYKQGQNQLYNE LNLGRREEYDVLDKRRGRDPEMGGKPRRKNPQEGLYNELQKDKMAEA YSEIGMKGERRRGKGHDGLYQGLSTATKDTYDALHMQALPPR | |
| CAR-T 20.12 | MALPVTALLLPLALLLHAARPEVQLQQSGAELVKPGASVKMSCKASGY TFTSYNMHWVKQTPGQGLEWIGAIYPGNGDTSYNQKFKGKATLTADK SSSTAYMQLSSLTSEDSADYYCARSNYYGSSYWFFDVWGAGTTVTVSS GGGGSGGGGSGGGGSDIVLTQSPAILSASPGEKVTMTCRASSSVNYMD WYQKKPGSSPKPWIYATSNLASGVPARFSGSGSGTSYSLTISRVEAEDA ATYYCQQWSFNPPTFGGGTKLEIKESKYGPPCPPCPAPEFLGGPSVFLFP PKPKDTLMISRTPEVTCVVVDVSQEDPEVQFNWYVDGVEVHNAKTKPR EEQFNSTYRVVSVLTVLHQDWLNGKEYKCKVSNKGLPSSIEKTISKAKG QPREPQVYTLPPSQEEMTKNQVSLTCLVKGFYPSDIAVEWESNGQPENN YKTTPPVLDSDGSFFLYSRLTVDKSRWQEGNVFSCSVMHEALHNHYTQ KSLSLSLGKIYIWAPLAGTCGVLLLSLVITLYCKRGRKKLLYIFKQPFMR PVQTTQEEDGCSCRFPEEEEGGCELRVKFSRSADAPAYKQGQNQLYNE LNLGRREEYDVLDKRRGRDPEMGGKPRRKNPQEGLYNELQKDKMAEA YSEIGMKGERRRGKGHDGLYQGLSTATKDTYDALHMQALPPR | 31 |
| CAR-T 20.13 | MALPVTALLLPLALLLHAARPQVQLVQSGAEVKKPGSSVKVSCKASGY AFSYSWINWVRQAPGQGLEWMGRIFPGDGDTDYNGKFKGRVTITADK STSTAYMELSSLRSEDTAVYYCARNVFDGYWLVYWGQGTLVTVSSGG GGSGGGGSGGGGSDIVMTQTPLSLPVTPGEPASISCRSSKSLLHSNGITY LYWYLQKPGQSPQLLIYQMSNLVSGVPDRFSGSGSGTDFTLKISRVEAE DVGVYYCAQNLELPYTFGGGTKVEIKRTVESKYGPPCPPCPAPEFLGGP SVFLFPPKPKDTLMISRTPEVTCVVVDVSQEDPEVQFNWYVDGVEVHN AKTKPREEQFNSTYRVVSVLTVLHQDWLNGKEYKCKVSNKGLPSSIEK TISKAKGQPREPQVYTLPPSQEEMTKNQVSLTCLVKGFYPSDIAVEWES NGQPENNYKTTPPVLDSDGSFFLYSRLTVDKSRWQEGNVFSCSVMHEA LHNHYTQKSLSLSLGKIYIWAPLAGTCGVLLLSLVITLYCKRGRKKLLYI FKQPFMRPVQTTQEEDGCSCRFPEEEEGGCELRVKFSRSADAPAYKQG | 32 |
| | QNQLYNELNLGRREEYDVLDKRRGRDPEMGGKPRRKNPQEGLYNELQ KDKMAEAYSEIGMKGERRRGKGHDGLYQGLSTATKDTYDALHMQAL PPR | |
| CAR-T 20.14 | MALPVTALLLPLALLLHAARPEVQLVESGGGLVQPGRSLRLSCAASGFT FNDYAMHWVRQAPGKGLEWVSTISWNSGSIGYADSVKGRFTISRDNA KKSLYLQMNSLRAEDTALYYCAKDIQYGNYYYGMDVWGQGTTVTVS SGGGGSGGGGSGGGGSEIVLTQSPATLSLSPGERATLSCRASQSVSSYLA WYQQKPGQAPRLLIYDASNRATGIPARFSGSGSGTDFTLTISSLEPEDFA VYYCQQRSNWPITFGQGTRLEIKESKYGPPCPPCPAPEFLGGPSVFLFPP KPKDTLMISRTPEVTCVVVDVSQEDPEVQFNWYVDGVEVHNAKTKPR EEQFNSTYRVVSVLTVLHQDWLNGKEYKCKVSNKGLPSSIEKTISKAKG QPREPQVYTLPPSQEEMTKNQVSLTCLVKGFYPSDIAVEWESNGQPENN YKTTPPVLDSDGSFFLYSRLTVDKSRWQEGNVFSCSVMHEALHNHYTQ KSLSLSLGKIYIWAPLAGTCGVLLLSLVITLYCKRGRKKLLYIFKQPFMR PVQTTQEEDGCSCRFPEEEEGGCELRVKFSRSADAPAYKQGQNQLYNE LNLGRREEYDVLDKRRGRDPEMGGKPRRKNPQEGLYNELQKDKMAEA YSEIGMKGERRRGKGHDGLYQGLSTATKDTYDALHMQALPPR | 33 |
| CAR-T 20.15 | MALPVTALLLPLALLLHAARPGDVMGVDSGGGLVQPGGSRKLSCAAP GFTFSSFGMHWVRQAPEKGLEWVAYISSPSSTLHYADRVKGRFTISRDN PKNTLFLQMKLPSLCYGLLGPRDHVHRLLKGGGGSGGGGSGGGGSDIQ LTQSPAILSASPGEKVTMTCRASSSLSFMHWYQQKPGSSPKPWIYATSN LASGVPARFSGSGSGTSYSLTISTVEAEDAASYFCHQWSSNPLTFGAGT KLEIESKYGPPCPPCPAPEFLGGPSVFLFPPKPKDTLMISRTPEVTCVVVD VSQEDPEVQFNWYVDGVEVHNAKTKPREEQFNSTYRVVSVLTVLHQD WLNGKEYKCKVSNKGLPSSIEKTISKAKGQPREPQVYTLPPSQEEMTKN QVSLTCLVKGFYPSDIAVEWESNGQPENNYKTTPPVLDSDGSFFLYSRL TVDKSRWQEGNVFSCSVMHEALHNHYTQKSLSLSLGKIYIWAPLAGTC GVLLLSLVITLYCKRGRKKLLYIFKQPFMRPVQTTQEEDGCSCRFPEEE GGCELRVKFSRSADAPAYKQGQNQLYNELNLGRREEYDVLDKRRGRD PEMGGKPRRKNPQEGLYNELQKDKMAEAYSEIGMKGERRRGKGHDGL YQGLSTATKDTYDALHMQALPPR | 34 |
| CAR-T 20.16 | MALPVTALLLPLALLLHAARPQVQLQQPGAELVKPGASVKMSCKASG YTFTSYNMHWVKQTPGRGLEWIGAIYPGNGDTSYNQKFKGKATLTAD KSSSTAYMQLSSLTSEDSAVYYCARSTYYGGDWYFNVWGAGTTVTVS AGGGGSGGGGSGGGGSQIVLSQSPAILSASPGEKVTMTCRASSSVSYIH | 35 |
| | WFQQKPGSSPKPWIYATSNLASGVPVRFSGSGSGTSYSLTISRVEAEDA ATYYCQQWTSNPPTFGGGTKLEIKESKYGPPCPPCPAPEFLGGPSVFLFP PKPKDTLMISRTPEVTCVVVDVSQEDPEVQFNWYVDGVEVHNAKTKPR EEQFNSTYRVVSVLTVLHQDWLNGKEYKCKVSNKGLPSSIEKTISKAKG QPREPQVYTLPPSQEEMTKNQVSLTCLVKGFYPSDIAVEWESNGQPENN YKTTPPVLDSDGSFFLYSRLTVDKSRWQEGNVFSCSVMHEALHNHYTQ KSLSLSLGKIYIWAPLAGTCGVLLLSLVITLYCKRGRKKLLYIFKQPFMR PVQTTQEEDGCSCRFPEEEEGGCELRVKFSRSADAPAYKQGQNQLYNE LNLGRREEYDVLDKRRGRDPEMGGKPRRKNPQEGLYNELQKDKMAEA YSEIGMKGERRRGKGHDGLYQGLSTATKDTYDALHMQALPPR | |
| CAR-T 20.17 | MALPVTALLLPLALLLHAARPEVQLQQSGAELVKPGASVKMSCKASGY TFTSYNMHWVKQTPGQGLEWIGAIYPGNGDTSYNQKFKGKATLTADK SSSTAYMQLSSLTSEDSADYYCARSNYYGSSYWFFDVWGAGTTVTVSS GGGGSGGGGSGGGGSDIVLTQSPAILSASPGEKVTMTCRASSSVNYMD WYQKKPGSSPKPWIYATSNLASGVPARFSGSGSGTSYSLTISRVEAEDA ATYYCQQWSFNPPTFGGGTKLEIKESKYGPPCPPCPAPEFEGGPSVFLFP PKPKDTLMISRTPEVTCVVVDVSQEDPEVQFNWYVDGVEVHNAKTKPR EEQFQSTYRVVSVLTVLHQDWLNGKEYKCKVSNKGLPSSIEKTISKAKG QPREPQVYTLPPSQEEMTKNQVSLTCLVKGFYPSDIAVEWESNGQPENN YKTTPPVLDSDGSFFLYSRLTVDKSRWQEGNVFSCSVMHEALHNHYTQ KSLSLSLGKFWVLVVVGGVLACYSLLVTVAFIIFWVRSKRSRLLHSDY MNMTPRRPGPTRKHYQPYAPPRDFAAYRSKRGRKKLLYIFKQPFMRPV QTTQEEDGCSCRFPEEEEGGCELRVKFSRSADAPAYKQGQNQLYNELN LGRREEYDVLDKRRGRDPEMGGKPRRKNPQEGLYNELQKDKMAEAYS EIGMKGERRRGKGHDGLYQGLSTATKDTYDALHMQALPPR | 36 |
| CAR-T 20.18 | MALPVTALLLPLALLLHAARPEVQLQQSGAELVKPGASVKMSCKASGY TFTSYNMHWVKQTPGQGLEWIGAIYPGNGDTSYNQKFKGKATLTADK SSSTAYMQLSSLTSEDSADYYCARSNYYGSSYWFFDVWGAGTTVTVSS GGGGSGGGGSGGGGSDIVLTQSPAILSASPGEKVTMTCRASSSVNYMD WYQKKPGSSPKPWIYATSNLASGVPARFSGSGSGTSYSLTISRVEAEDA ATYYCQQWSFNPPTFGGGTKLEIKESKYGPPCPPCPAPEFEGGPSVFLFP PKPKDTLMISRTPEVTCVVVDVSQEDPEVQFNWYVDGVEVHNAKTKPR EEQFQSTYRVVSVLTVLHQDWLNGKEYKCKVSNKGLPSSIEKTISKAKG QPREPQVYTLPPSQEEMTKNQVSLTCLVKGFYPSDIAVEWESNGQPENN YKTTPPVLDSDGSFFLYSRLTVDKSRWQEGNVFSCSVMHEALHNHYTQ | 37 |
| | KSLSLSLGKIYIWAPLAGTCGVLLLSLVITLYCKRGRKKLLYIFKQPFMR PVQTTQEEDGCSCRFPEEEEGGCELRVKFSRSADAPAYKQGQNQLYNE LNLGRREEYDVLDKRRGRDPEMGGKPRRKNPQEGLYNELQKDKMAEA YSEIGMKGERRRGKGHDGLYQGLSTATKDTY | |
| CAR-T 20.19 | MALPVTALLLPLALLLHAARPEVQLVESGGGLVQPGRSLRLSCAASGFT FNDYAMHWVRQAPGKGLEWVSTISWNSGSIGYADSVKGRFTISRDNA KKSLYLQMNSLRAEDTALYYCAKDIQYGNYYYGMDVWGQGTTVTVS SGGGGSGGGGSGGGGSEIVLTQSPATLSLSPGERATLSCRASQSVSSYLA WYQQKPGQAPRLLIYDASNRATGIPARFSGSGSGTDFTLTISSLEPEDFA VYYCQQRSNWPITFGQGTRLEIKESKYGPPCPPCPAPEFEGGPSVFLFPP KPKDTLMISRTPEVTCVVVDVSQEDPEVQFNWYVDGVEVHNAKTKPR EEQFQSTYRVVSVLTVLHQDWLNGKEYKCKVSNKGLPSSIEKTISKAKG QPREPQVYTLPPSQEEMTKNQVSLTCLVKGFYPSDIAVEWESNGQPENN YKTTPPVLDSDGSFFLYSRLTVDKSRWQEGNVFSCSVMHEALHNHYTQ KSLSLSLGKIYIWAPLAGTCGVLLLSLVITLYCKRGRKKLLYIFKQPFMR PVQTTQEEDGCSCRFPEEEEGGCELRVKFSRSADAPAYKQGQNQLYNE LNLGRREEYDVLDKRRGRDPEMGGKPRRKNPQEGLYNELQKDKMAEA YSEIGMKGERRRGKGHDGLYQGLSTATKDTYDALHMQALPPR | 38 |
| | | |

### Example 9 Phase I Clinical Trial of TN-OF-19 CAR-T cells

A Phase 1 trial was conducted in patients with relapsed/refractory non-Hodgkin lymphoma (R/R NHL) to assess the safety and efficacy of C-CAR039 (i.e., TN-OF-19 CAR-T cells, NCT04317885). Following apheresis to harvest T cells, C-CAR039 was manufactured and infused as a single intravenous dose after a standard 3-day cyclophosphamide/fludarabine conditioning regimen. C-CAR039 was manufactured in a serum free, semi-automated, and digitally closed system with median vein to vein time of 18 days. The manufacturing success rate was 100%. As of Aug 3, 2020, 16 patients were infused with C-CAR039 with a dose range of 1.0 x 10⁶ to 5.0 x 10⁶ CAR-T cells/kg. 14 patients had at least one-month evaluable safety data and 13 patients (11 DLBCL, 2 FL patients) had one-month or longer efficacy data.

The key eligibility criteria include: (1) 18-75 years of age; (2) r/r B-NHL including DLBCL, FL, MCL; (3) either CD19 or CD20 positive disease; (4) no prior anti-CD19 therapy; and (5) no active CNS involvement. The safety assessment includes the incidence and severity of treatment-emergent adverse events (CTCAE V5.0). The efficacy assessment includes the Lugano Classification 2014: ORR; DOR; PFS; OS.

As the data cut-off at Jan 31, 2021, 30 patients have been enrolled in four clinical centers; 28 patients have been dosed; The median vein-to-vein time was 19 days. 25 patients have 1-month data; 17 patients have 3-month data; 12 patients have 6-month data. 1 infused patient does not have evaluable disease activity at baseline.

The patients' baseline demographics and clinical characteristics prior to the start of our bispecific CAR treatment are shown in Table 3.

The median age of patients dosed was 54 years (range: 28-71 years).The median number of prior lines of therapy was 3 (range: 1-5 prior therapies). There were 5 (20%) patients had previous autologous stem cell transplantation (ASCT).

**Table 3 Summary of baseline clinical characteristics of patients**

| **Characteristic** | **N=25** | **Characteristic** | **N=25** |
|---|---|---|---|
| Median age, yrs (range) | 54 (28-71) | Median number of prior lines of therapy, n (range) | 3 (1-5) |
| • Age ≥ 65, n (%) | 8 (32.0) | •1, n (%) | 1 (4.0) |
| Male/Female, n | 18/7 | •2, n (%) | 9 (36.0) |
| NHL subtype, n (%) | | •3, n (%) | 3 (12.0) |
| • DLBCL | 12 (88.0) | •4, n (%) | 7 (28.0) |
| • PMBCL | 1 (4.0) | •5, n (%) | 5 (20.0) |
| • FL | 1 (4.0) | Previous ASCT, n (%) | 5 (20.0) |
| • tFL | 1 (4.0) | Best response as PD to last prior therapy, n (%) | 14 (56.0) |
| ECOG PS, n (%) | | Received bridging therapy, n (%) | 5 (20.0) |
| • 0 | 15 (60.0) | Median infusion dosage, ×10⁶/kg (range) | 2.5 (1.0-5.0) |
| • 1 | 10 (40.0) | | |
| IPI score 3/4, n (%) | 6 (24.0) | | |
| Ann Arbor stage III/IV, n (%) | 19 (76.0) | | |

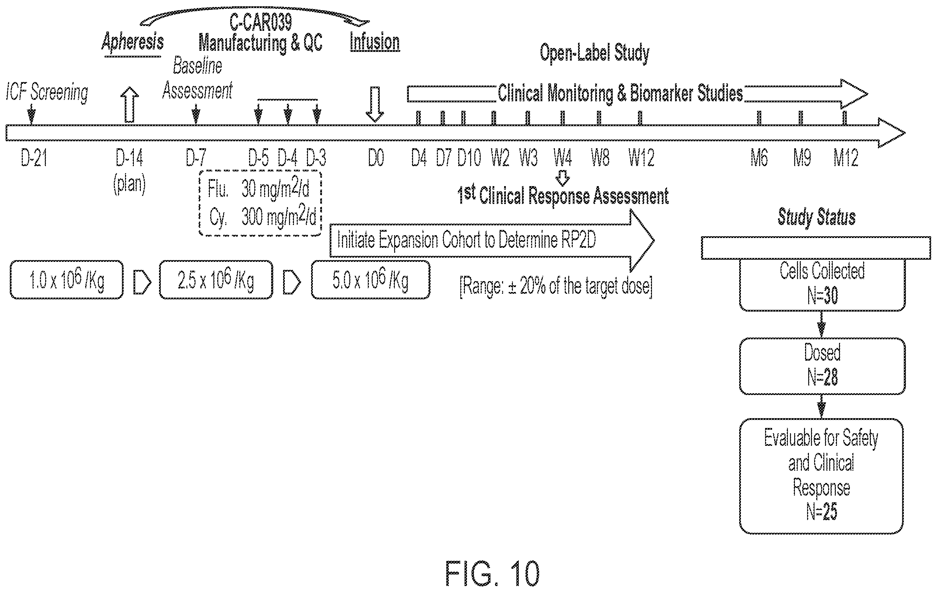

Figure 10 shows CAR039 r/r NHL study design and flow chart. Specifically, the patients were screened 21 days before the treatment (D-21). Qualified subjects were enrolled, and peripheral blood leukocytes collected. The collected peripheral blood leukocytes were used to produce the CAR-T cells. At -5 (D-5), -4 (D-4) and -3 (D-3) days before the CAR-T infusion, the patients received lymphodepletion pretreatment, including fludarabine (30 mg/m²/d, intravenous, once per day for three days), and cyclophosphamide (300 mg/m²/d, intravenous, once per day for three days). Approximately 72 hours after lymphodepletion, the patients were administered 1.0 x 10⁶, 2.5 x 10⁶ or 5.0 x 10⁶ CAR-T cells/kg body weight on day 0 (D0) as a single infusion. Follow-ups with the patients were carried out on day 4, day 7, day 10, week 2, week 3, week 4, week 8, week 12, month 6, month 9 and month 12 after the infusion. The first clinical response assessment was at week 4 after the CAR-T infusion.

The patients' adverse reactions (treatment-emergent adverse events, TEAE) were recorded (Table 4). There was only 1 (4%) grade 3 or greater cytokine release syndrome (CRS). Neurotoxicity was observed only in two patients (8.0%), with no grade 3 or greater neurotoxicity. Cytopenia, such as neutropenia and thrombocytopenia, was mostly related to the fludarabine/cyclophosphamide (Cy/Flu) lymphodepletion. The cytopenia was also reversible. These demonstrated that the bispecific CAR had an excellent safety profile.

**Table 4 Summary of Treatment-emergent Adverse Events (AEs)**

| **AEs* (≥25%) n (%)** | **Any Grade (N=25)** | **Grade ≥3 (N=25)** |
|---|---|---|
| Neutropenia | 25 (100) | 22 (88.0) |
| Thrombocytopenia | 13 (52.0) | 4 (16.0) |
| Anemia | 24 (96.0) | 8 (40.0) |
| Infection | 13 (52.0) | 0 (0) |

| | | |
|---|---|---|
| * CTCAE V5.0. | | |

**Table 5 Safety profile**

| CRS & Neurotoxicity** | | Any Grade (N=25) | Grade ≥3 (N=25) |
|---|---|---|---|
| CRS, n (%) | | 24 (96.0) | 1 (4) |
| | • Median days to onset, d (range) | 3 (0-10) | |
| | **•** Median days to resolution, d (range) | 4 (1-25) | |
| | • Treated with Tocilizumab, n (%) | 4 (16.0) | |
| | • Treated with Steroids, n (%) | 2 (8.0) | |
| Neurotoxicity, n (%) | | 2 (8.0) | 0 (0) |
| | • Day to onset, d | 16 (4-28) | |
| | • Day to resolution, d | NA | |
| | **•** Treated with Steroids, n (%) | 1 (4.0) | |
| ICU admissions, n (%) | | 0 | |

| | | | |
|---|---|---|---|
| ** ASTCT Consensus Grading for Cytokine Release Syndrome and Neurologic Toxicity Associated with Immune Effector Cells 2019. | | | |

**Table 6 CRS & Neurotoxicity**

| CRS & Neurotoxicity | | All (N=25) | 1.0 MPK (N=4) | 2.0/2.5 MPK (N=14) | 4.0/5.0 MPK (N=7) |
|---|---|---|---|---|---|
| CRS, n (%) | | 24 (96.0) | 4 (100) | 13 (92.9) | 7 (100) |
| | • Grade 1/2/3/4/5, n | 18/5/1/0/0 | 3/1/0/0/0 | 10/2/1/0/0 | 5/2/0/0/0 |
| | • Median days to onset, d (range) | 3 (0-10) | 7 (2-10) | 4 (1-10) | 1 (0-9) |
| | **•** Median days to resolution, d (range) | 4 (1-25) | 2.5 (1-7) | 4 (1-25) | 6 (1-15) |
| | • Treated with vasopressors, % | 1 (4.0) | 0 (0) | 1 (7.1) | 0 (0) |
| | • Treated with Tocilizumab, % | 4 (16.0) | 0 (0) | 2 (14.3) | 2 (28.6) |
| | • Treated with steroids, % | 2 (8.0) | 0 (0) | 2 (14.3) | 0 (0) |
| Neurotoxicity*, n (%) | | 2 (8.0) | 0 (0) | 0 (0) | 2 (28.6) |
| Grade 1/2/3/4/5, n | | 2/0/0/0/0 | 0/0/0/0/0 | 0/0/0/0/0 | 2/0/0/0/0 |
| Median days to onset, d (range) | | 16 (4-28) | NA | NA | |
| Median days to resolution*, d (range) | | NA | NA | NA | NA |
| Treated with steroids, % | | 1 (4.0) | NA | NA | 1 (4.0) |
| ICU admissions, n (%) | | 0 | 0 | 0 | 0 |

| | | | | | |
|---|---|---|---|---|---|
| * 1 patient has not resolved by the cutoff date. | | | | | |

C-CAR039 treatment was well tolerated with 1 grade 3 CRS in 25 patients and 2 Grade 1 neurotoxicity events. Cytopenias were mostly related to the conditioning regimen (Cy/Flu lymphodepletion) and were common and reversible.

At the one-month evaluation, 12/13 patients showed clinical improvement (overall response rate or ORR=92%) and 11/11 of DLBCL patients responded to the treatment (ORR=100%). Median follow-up was 70 days (range: 35-257 days). The best overall response (BOR) includes 10 complete responses (CRs) and 2 partial responses (PRs).

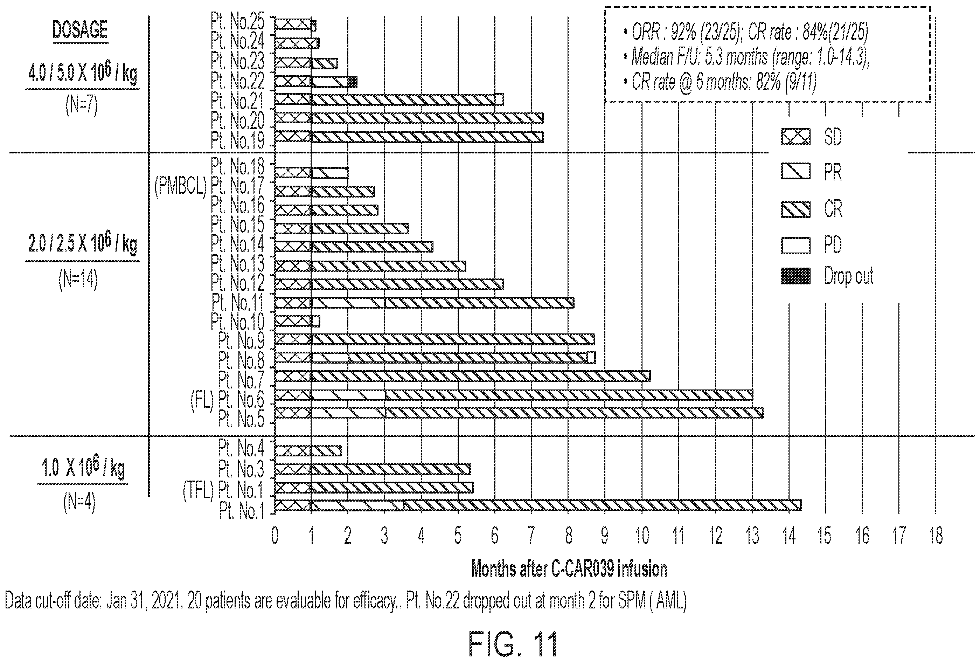

Figure 11 shows the summary of C-CAR039 clinical results. There were three (3) dosages administered: (1) 4.0x10⁶ or 5.0x10⁶ /kg (N=7); (2) 2.0x10⁶ or 2.5x10⁶ /kg (N=14); and (3) 1.0x10⁶ /kg (N=4).

After approximately 14 months, the ORR was 92% (23 out of 25 patients); CR was 84% (21 out of 25 patients). The CR rate at 6 months was 82% (9 out of 11 patients). Median follow-up (F/U) was 5.3 months (range: 1.0 - 14.3 months).

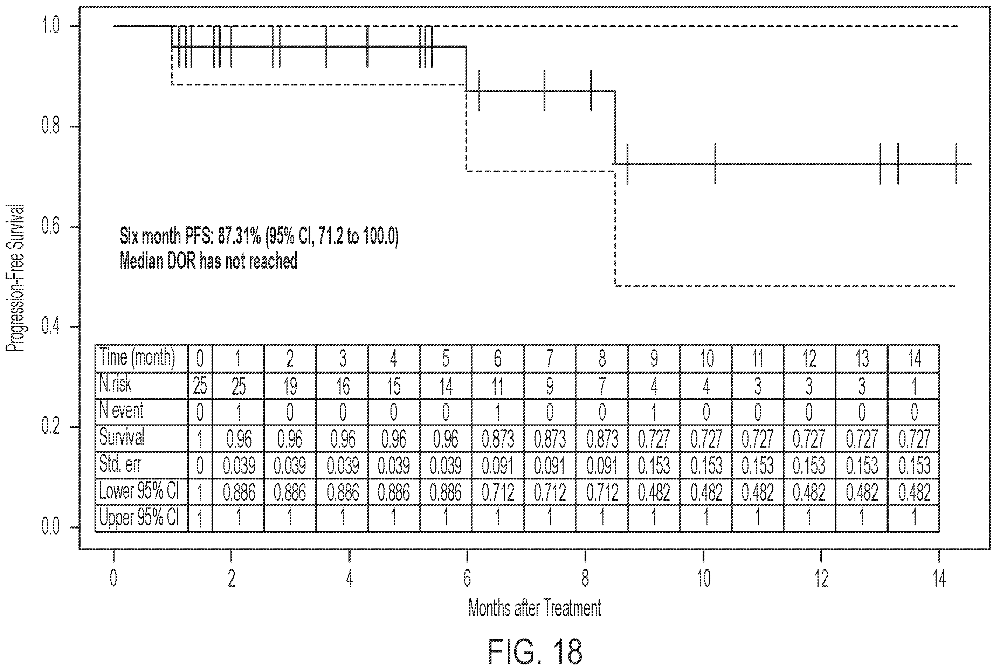

Figure 18 shows the Kaplan Meyer estimation of PFS for C-CAR039. The 6-month PFS is 87.31%, with 95% confidence intervals (CIs) of 71.2 to 100.0. Median duration of response (DOR) has not reached.

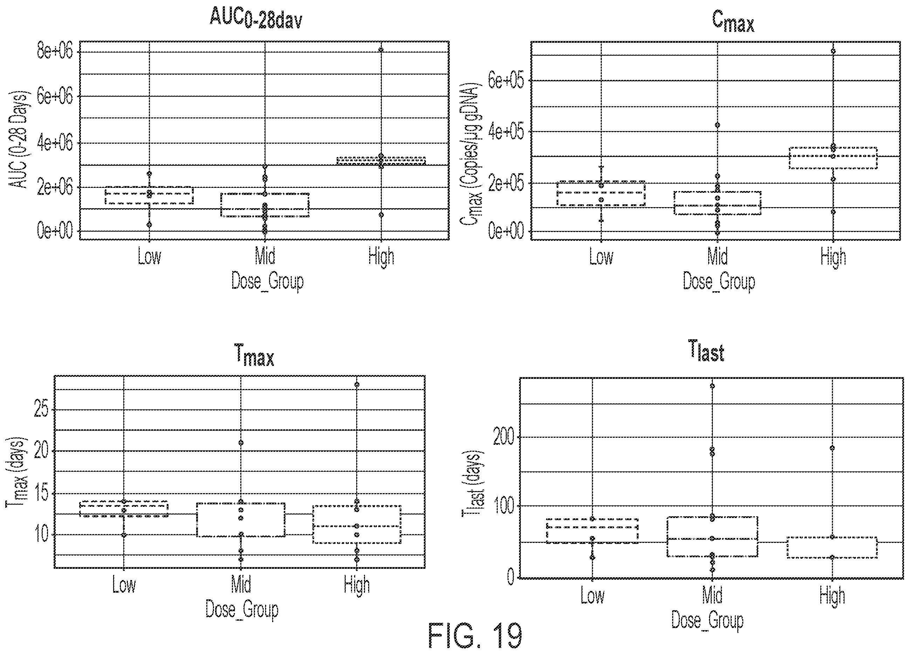

Figure 19 shows the PK/PD profile of C-CAR039. There were no statistic differences of PK profiles between the different dose groups.

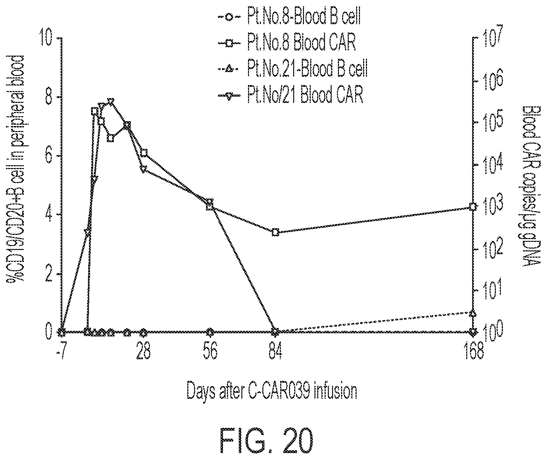

Figure 20 shows the PK/PD profile (CAR-T expansion and B cell depletion) in the peripheral blood of the relapsed patients.

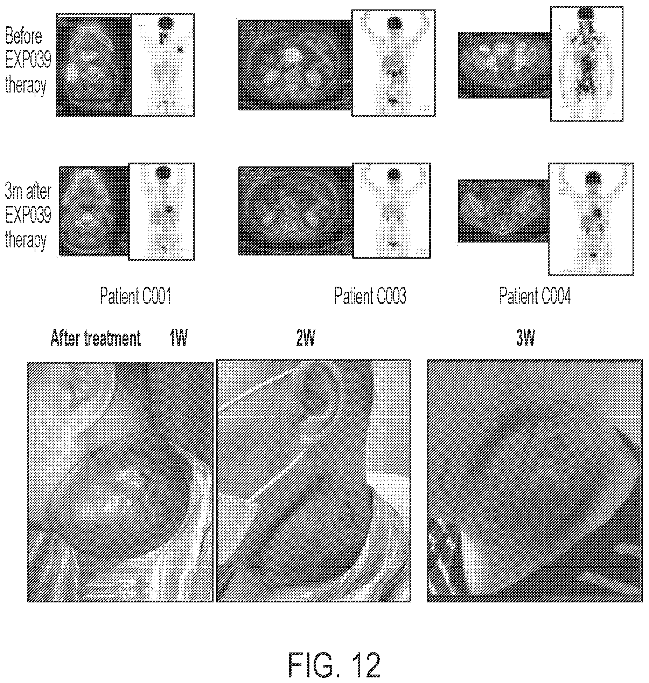

Figure 12 shows examples of patients before and post C-CAR039 treatment. It clear shows that the tumor lesions decreased significantly in size three months after the CAR T treatment.

### Example 10 PK profile of C-CAR039

C-CAR039 proliferation and expansion in the peripheral blood positively correlated with tumor regression. Positive early trend of correlation between C-CAR039 AUC (0-28 day) and Cmax and clinical response is observed.

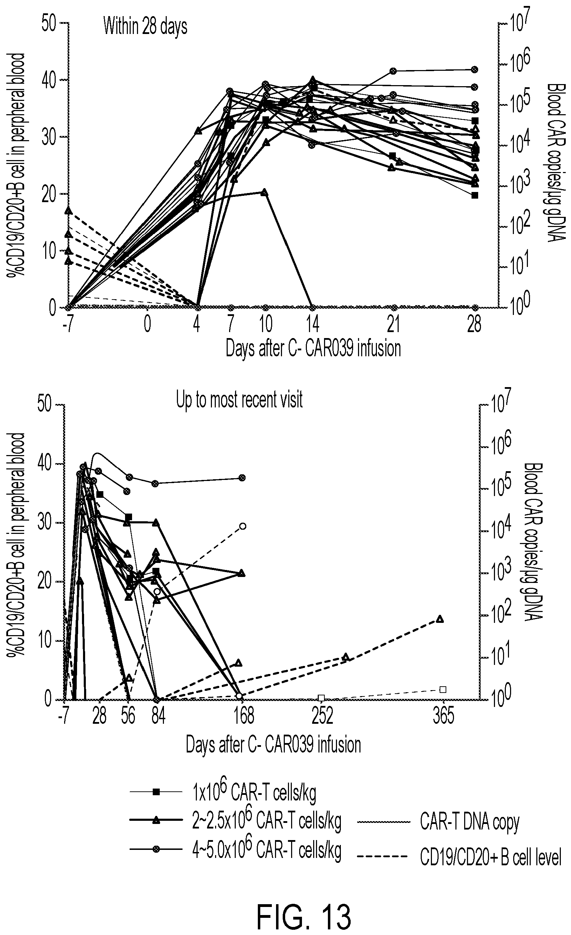

Figure 13 shows C-CAR039 proliferation and expansion in patient's blood. The results showed that C-CAR008 cells expanded effectively after injection.

Table 7 shows the CD19/CD20 expression in relapsed patients.

**Table 7 CD19/CD20 expression tested in tumor tissues by IHC**

| | Before treatment | Relapsing after CCAR066 treatment |
|---|---|---|
| Patient (Pt.) No. 8 | CD19(+) CD20(+) | CD19(+) CD20(+) |
| Pt. No. 21 | CD19(+) CD20(+) | CD19(-) CD20(+) |

**Table 8. The relationships of PK profile (AUC_{0∼28day}, Cmax, Tmax, Tlast), CAR-T cell dose and clinical responses.**

| Subj ID | Dose (10^6 CAR-T/kg) | Best Response | AUC0~28 day | Cmax | Tmax | Tlast | Follow up(day) |
|---|---|---|---|---|---|---|---|
| Pt. No.8 | 3 | SD | 7,530 | 708 | 10 | 60 | 60+ |
| Pt. No.13 | 3 | CR | 201,811 | 30,290 | 10 | 28 | 28+ |
| Pt. No.7 | 4 | UE | 223,932 | 57,478 | 31 | 60 | 60+ |
| Pt. No.9 | 3 | PR | 537,713 | 38,316 | 7 | 57 | 57+ |
| Pt. No.4 | 3 | PR | 607,007 | 89,655 | 12 | 84 | 84+ |
| Pt. No.12 | 5 | CR | 727,947 | 83,494 | 8 | 28 | 28+ |
| Pt. No2 | 2 | CR | 747,101 | 114,041 | 14 | 187 | 187+ |
| Pt. No.3 | 2 | CR | 1,154,844 | 139,497 | 10 | 182 | 182+ |
| Pt. No.5 | 3 | CR | 1,673,877 | 183,427 | 8 | 85 | 85+ |
| Pt. No.6 | 3 | CR | 2,437,574 | 223,644 | 13 | 55 | 55+ |
| Pt. No1 | 1 | CR | 2,580,272 | 259,462 | 14 | 104 | 257+ |
| Pt.No.10 | 2 | CR | 2,953,074 | 308,343 | 11 | 55 | 55+ |
| Pt. No.14 | 4 | CR | 3,143,662 | 302,300 | 14 | 28 | 28+ |
| Pt. No.11 | 5 | CR | 8,117,064 | 715,187 | 28 | 28 | 28+ |

C-CAR039 proliferation & expansion in the peripheral blood positively correlated with tumor regression. Positive early trend of correlation between C-CAR039 AUC (0-28 day) and Cmax and clinical response is observed.

**Table 9 SCHOLAR-1: Outcome of refractory DLBCL with non-CAR T-cell therapy (pooled retrospective analysis)**

| Outcome | | MDACC (n=165) | IA/MC (n=82) | LY.12 (CCTG) (n=106) | CORAL (n=170) | Pooled (n=523) |
|---|---|---|---|---|---|---|
| Response rate, % | | 20 | 26 | 26 | 31 | 26 |
| | • CR | 7 | 7 | 2 | 15 | 7 |
| | • PR | 13 | 18 | 25 | 16 | 18 |
| Response rate (category), % | | | | | | |
| | • Primary refractory | NR | 25 | 27 | 10 | 20 |
| | • Refractory to ≥ 2nd-line therapy | 20 | 21 | 20 | 40 | 26 |
| | • Relapse ≤ 12 mos post-ASCT | 19 | 35 | NR | 39 | 34 |
| Median OS, mos | | 6.6 | 5.0 | 6.6 | 6.5 | 6.3 |

| | | | | | | |
|---|---|---|---|---|---|---|
| Crump. Blood. 2017; 130:1800. | | | | | | |

**Table 10 C-CAR039 Compared with commercial anti-CD19 CART products**

| | ZUMA-1[1] (n=101) | JULIET[2] (n=111) | TRANSCEND NHL 001[3] (n=269**) | C-CAR039 (n=25) |
|---|---|---|---|---|
| Median age, yrs (range) | 58 (23-76) | 56 (22-76) | 63 (54-70) | 54 (28-71) |
| DLBCL (including HGBL), % | 76 | 79 | 64 | 88 |
| ECOG 0/1, % | 100 | 100 | 99 | 100 |
| ≥ 3 prior therapies, % | 69 | 51 | 51 | 60 |
| IPI 3/4, % | 48 | - | - | 24 |
| Ann Arbor stage III/IV, % | 85 | 76 | - | 76 |
| Prior ASCT, % | 21 | 49 | 33 | 20 |
| Best response as PD or SD to last prior therapy, % | 66(PD) | 55 | 75 (PD,SD,PR) | 80 |
| ORR, % | 82 (Best) | 52 (Best) | 73 (Best) | 92 (Best) |
| CR, % | 58 (Best) | 40 (Best) | 53 (Best) | 84 (Best) |
| Grade ≥ 3 CRS/NEs, % | 13*/31† | 22‡/12† | 2†/10 | 4/0 |
| Tocilizumab/steroid use, % | 43/27 | 14/10 | 18/10 | 14/12 |

| | | | | |
|---|---|---|---|---|
| **256 included in the efficacy-evaluable set. *Per Lee scale. †Per CTCAE V4.03. ‡Per Penn Scale. §Per ASTCT). 1. Neelapu. NEJM. 2017; 377:2531. 2. Schuster. NEJM. 2019; 380:45. 3. Schuster. NEJM. 2019; 380:45 | | | | |

**Table 11 C-CAR039 Compared to other CD19/CD20 bi-specific CAR-T products**

| | Nirav N. Shah[1] (n=22) | Tong C[2] (n=28) | C-CAR039 (n=25) |
|---|---|---|---|
| Median age, yrs (range) | 57 (38-72) | ≥60 (25%) | 54 (28-71) |
| DLBCL (including HGBL), % | 50 | 57 | 88 |
| ECOG 0/1, % | 100 | 54 | 100 |
| ≥ 3 prior therapies, % | - | 79 | 60 |
| Ann Arbor stage III/IV, % | - | 82 | 76 |
| Prior ASCT, % | 37 | 18 | 20 |
| Best response as PD or SD to last prior therapy, % | 82 | 86 | 80 |
| ORR (best), % | 82 | 79 | 92 |
| CR (best), % | 64 | 71 | 84 |
| Grade ≥ 3 CRS/NEs, % | 5/14 | 14/0 | 4/0 |
| Tocilizumab/steroid use, % | - | - | 14/12 |

C-CAR039 shows promising efficacy and a favorable safety profile in the clinical trial in patients with r/r NHL. The efficacy compares favorably to anti-CD19 CAR-T therapies.

### Example 11 Phase I Clinical Trial of C-CAR066 (CAR-T20.19 (OF))

Relapse due to loss of the CD19 targeted epitope presents a therapeutic challenge of CD19 CAR-T therapy. These patients universally have poor outcomes. CD20 is a proven therapeutic target for B-Cell Non-Hodgkin Lymphomas (B-NHL), supported by previously approved and widely used monoclonal antibody therapy.C-CAR066 is a novel 2nd generation chimeric antigen receptor T (CAR-T) therapy. Preclinical studies suggest that C-CAR066 (derived from scFVs of Ofatumumab) has superior anti-tumor activity compared to CAR-Ts derived from scFVs of Leu16, Rituximab, and Obinutuzumab,

NCT04036019 is a single arm, single-center, non-randomized phase I clinical trial to evaluate the safety and efficacy of C-CAR066 in subjects with r/r B cell lymphoma who were previously treated with anti-CD19 CAR-T therapy. The primary objective of the study is to evaluate incidence and severity of treatment emergent adverse events. The secondary objectives include determining overall response rate (ORR), PFS, and OS. C-CAR066 is manufactured in a serum free, semi-automated, and digitally closed system. C-CAR066 is administered to patients as a single intravenous dose after a standard 3-day cyclophosphamide/fludarabine conditioning regimen.

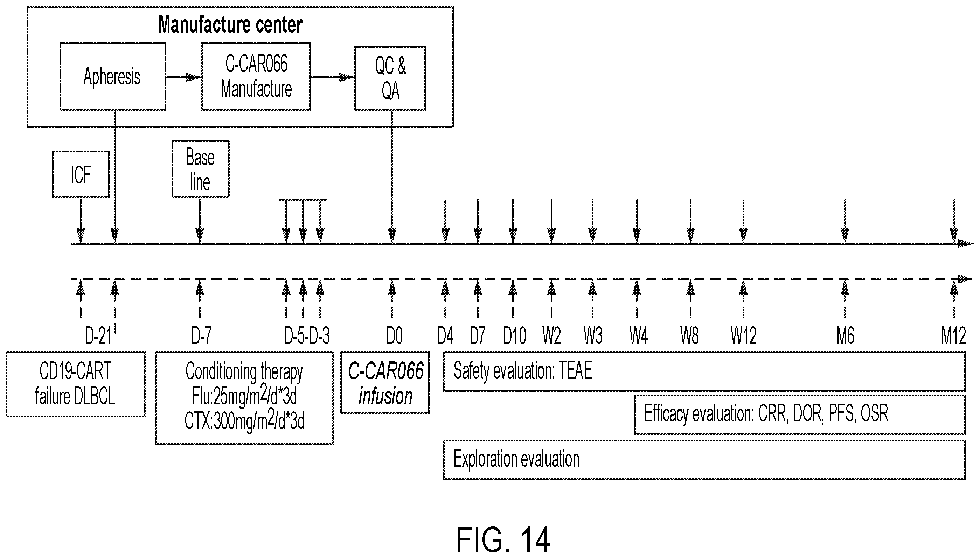

Figure 14 shows the C-CAR066-NHL study design.

As of Aug 3, 2020, 7 patients (all DLBCL) were enrolled and infused with C-CAR039 with a dose range of 2.0 x 10⁶ to 5.0 x 10⁶ CAR-T cells. The manufacturing success rate was 100%. All patients had relapsed after anti-CD19 CAR-T treatment, only one of the patients had achieved CR following anti-CD-19 CAR-T therapy

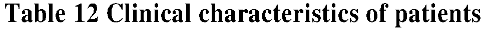

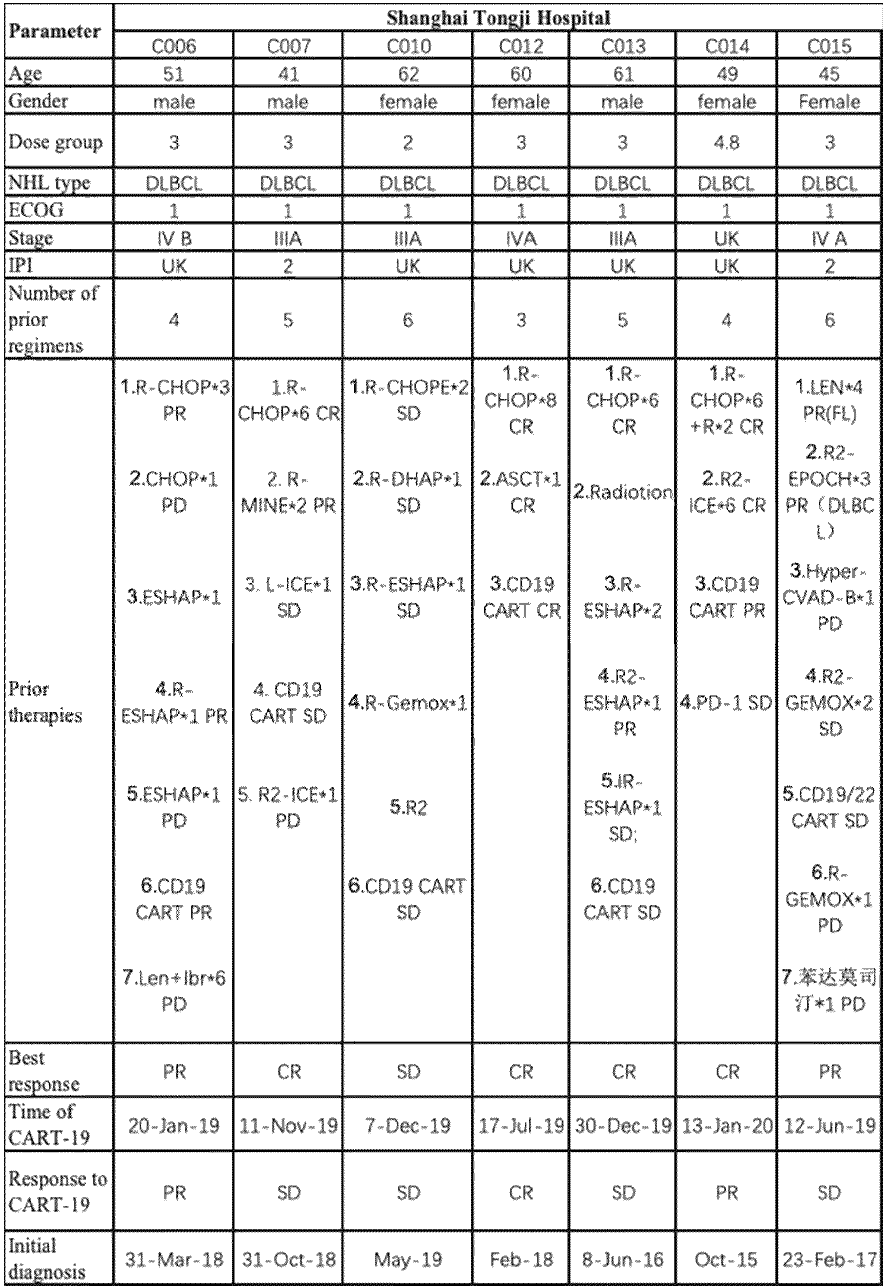

C-CAR066 treatment was well tolerated with reversible grade 1~2 CRS in six patients, grade 3 CRS in another patient, and no neurotoxicity events. 6/7 patients showed clinical improvement (best overall response rate, ORR = 85.7%). The best overall responses include 3 CR and 3PR. All patients responded to C-CAR066 treatment and showed different degrees of tumor regression (45-100%).

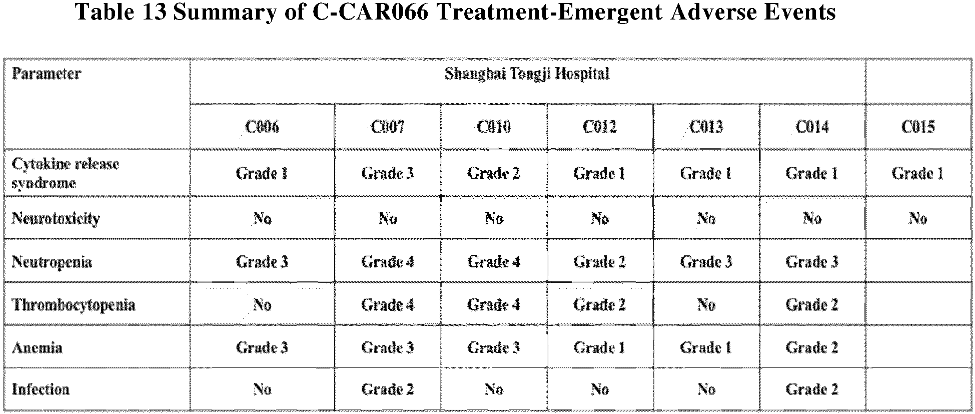

6/7 patients showed clinical improvement (best overall response rate, ORR = 85.7%). The best overall responses include 3 CR and 3PR. All patients responded to C-CAR066 treatment and showed different degrees of tumor regression (45-100%).

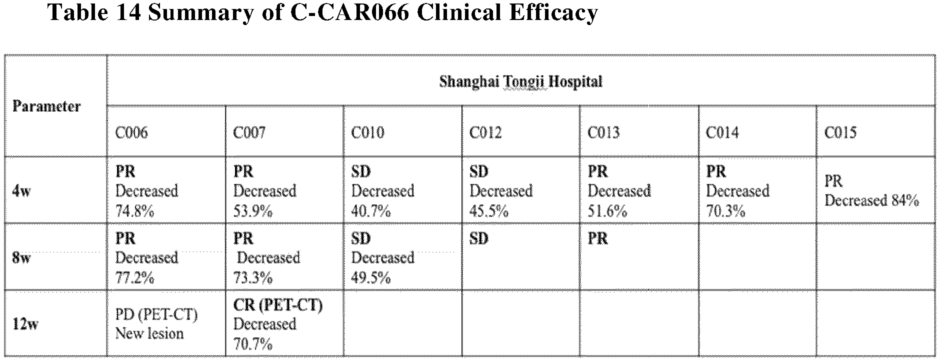

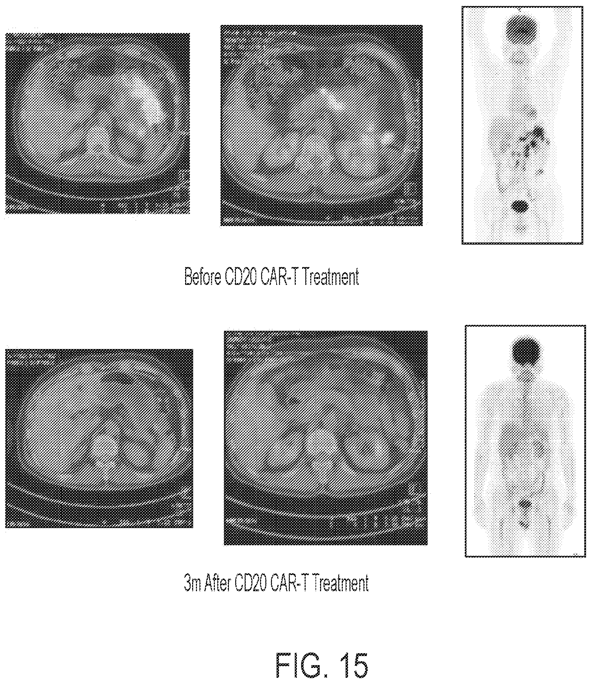

Figure 15 shows an example of PET-CT of a patient before and after treated with C-CAR066.

C-CAR066 has a favorable safety profile and shows promising early efficacy in patients with r/r NHL following CD19 CAR-T therapy. It shows that C-CAR066 has a different mechanism of action compared to anti-CD-19 CAR-T therapy. By targeting both CD20 and CD19 tumor antigens might lead to superior clinical benefit to targeting either CD19 or CD20 alone in B-cell malignancy patients.

## Claims

1. A bispecific chimeric antigen receptor (CAR) targeting CD19 and CD20, wherein the CAR comprises the amino acid sequence set forth in SEQ ID NO. 16.

2. An immune cell expressing the bispecific CAR of claim 1.

3. The immune cell of claim 2, which is a T cell.

4. The immune cell of claim 2, which is a natural killer (NK) cell.

5. A nucleic acid encoding the bispecific CAR of claim 1.

6. A vector comprising the nucleic acid of claim 5.

7. The immune cell of any of claims 2 to 4, for use in treating cancer in a subject.

8. The immune cell for use according to claim 7, wherein the immune cell is allogeneic with respect to the subject.

9. The immune cell for use according to claim 7, wherein the immune cell is autologous with respect to the subject.

10. The immune cell for use according to any of claims 7 to 9, wherein the cancer is a hematologic cancer or a B-cell malignancy.

11. The immune cell for use according to claim 10, wherein the cancer is Hodgkin's lymphoma, non-Hodgkin's lymphoma, leukemia, and/or multiple myeloma, or acute myeloid leukemia (AML), multiple myeloma (MM), chronic lymphocytic leukemia (CLL), chronic myelogenous leukemia, acute lymphoblastic leukemia (ALL), diffuse large B cell lymphoma (DLBCL), or combinations thereof.

12. A pharmaceutical composition comprising a T cell expressing the bispecific CAR of claim 1, wherein the composition further comprises one or more pharmaceutically or physiologically acceptable carriers, diluents or excipients.

13. The pharmaceutical composition of claim 12, for use in treating a cancer, wherein the composition is to be administered by i.v. injection.

## Patentansprüche

1. Ein bispezifischer, chimärer Antigenrezeptor (CAR), der gegen CD19 und CD20 gerichtet ist, wobei der CAR die Aminosäuresequenz wie in SEQ ID NO: 16 beschrieben, umfasst.

2. Eine Immunzelle, die den bispezifischen CAR nach Anspruch 1 exprimiert.

3. Die Immunzelle nach Anspruch 2, die eine T-Zelle ist.

4. Die Immunzelle nach Anspruch 2, die eine natürliche Killer (NK)-Zelle ist.

5. Eine Nukleinsäure, die den bispezifischen CAR nach Anspruch 1 kodiert.

6. Ein Vektor umfassend die Nukleinsäure nach Anspruch 5.

7. Die Immunzelle nach einem der Ansprüche 2 bis 4 zur Verwendung in der Behandlung von Krebs in einem Subjekt.

8. Die Immunzelle zur Verwendung nach Anspruch 7, wobei die Immunzelle allogen ist in Bezug auf das Subjekt.

9. Die Immunzelle zur Verwendung nach Anspruch 7, wobei die Immunzelle autolog ist in Bezug auf das Subjekt.

10. Die Immunzelle zur Verwendung nach einem der Ansprüche 7 bis 9, wobei der Krebs ein hämatologischer Krebs oder eine B-Zell-Malignität ist.

11. Die Immunzelle zur Verwendung nach Anspruch 10, wobei der Krebs Hodgkin-Lymphom, Non-Hodgkin-Lymphom, Leukämie, und/oder multiples Myelom oder akute myeloische Leukämie (AML), multiples Myelom (MM), chronische lymphatische Leukämie (CLL), chronische myeloische Leukämie (CML), akute lymphatische Leukämie (ALL), diffuses großzelliges B-Zell-Lymphom (DLBCL) oder Kombinationen davon ist.

12. Eine pharmazeutische Zusammensetzung umfassend eine T-Zelle, die den bispezifischen CAR nach Anspruch 1 exprimiert, wobei die Zusammensetzung ferner ein oder mehrere pharmazeutisch oder physiologisch verträgliche Träger, Verdünnungsmittel oder Hilfsstoffe umfasst.

13. Die pharmazeutische Zusammensetzung nach Anspruch 12 zur Verwendung in der Behandlung von Krebs, wobei die Zusammensetzung mittels i.v. Injektion verabreicht wird.

## Revendications

1. Récepteur d'antigène chimérique (CAR) bispécifique ciblant CD19 et CD20, lequel CAR comprend la séquence d'acides aminés présentée dans la SEQ ID NO : 16.

2. Cellule immunitaire exprimant le CAR bispécifique de la revendication 1.

3. Cellule immunitaire selon la revendication 2, qui est une cellule T.

4. Cellule immunitaire selon la revendication 2, qui est une cellule tueuse naturelle (NK).

5. Acide nucléique codant pour le CAR bispécifique de la revendication 1.

6. Vecteur comprenant l'acide nucléique de la revendication 5.

7. Cellule immunitaire selon l'une quelconque des revendications 2 à 4, pour une utilisation dans le traitement d'un cancer chez un sujet.

8. Cellule immunitaire pour une utilisation selon la revendication 7, laquelle cellule immunitaire est allogénique vis-à-vis du sujet.

9. Cellule immunitaire pour une utilisation selon la revendication 7, laquelle cellule immunitaire est autologue vis-à-vis du sujet.

10. Cellule immunitaire pour une utilisation selon l'une quelconque des revendications 7 à 9, dans laquelle le cancer est un cancer hématologique ou une malignité à cellules B.

11. Cellule immunitaire pour une utilisation selon la revendication 10, dans laquelle le cancer est un lymphome hodgkinien, un lymphome non hodgkinien, une leucémie, et/ou un myélome multiple, ou une leucémie myéloïde aiguë (AML), un myélome multiple (MM), une leucémie lymphoïde chronique (CLL), une leucémie myélocytaire chronique, une leucémie lymphoblastique aiguë (ALL), un lymphome diffus à grosses cellules B (DLBCL), ou des combinaisons de ceux-ci.

12. Composition pharmaceutique comprenant une cellule T exprimant le CAR bispécifique de la revendication 1, laquelle composition comprend en outre un ou plusieurs véhicules, diluants ou excipients pharmaceutiquement ou physiologiquement acceptables.

13. Composition pharmaceutique selon la revendication 12, pour une utilisation dans le traitement d'un cancer, laquelle composition est destinée à être administrée par injection i.v.
